# EUROPÄISCHE PATENTANMELDUNG

(11) **EP 4 846 967 A1**
(43) Veröffentlichungstag der Anmeldung: **02.12.2026**
(21) Anmeldenummer: 26181489.1
(22) Anmeldetag: 28.05.2026
(51) Int. Cl.: A61B 18/14, A61B 18/00, A61B 18/12

(54) **CHIRURGISCHES BIPOLAR-MAULTEILWERKZEUG, CHIRURGISCHES BIPOLAR-INSTRUMENT UND HERSTELLUNGSVERFAHREN**

(30) Priorität: 28.05.2025 DE 102025121165
(71) Anmelder: Karl Storz SE & Co. KG, 78532 Tuttlingen (DE)
(72) Erfinder: STEFAN, Jochen, 78532 Tuttlingen (DE)

(57) **Zusammenfassung**

Die vorliegende Erfindung stellt chirurgisches Bipolar-Maulteilwerkzeug (1), ein Verfahren zu seiner Herstellung und ein entsprechendes Bipolar-Instrument (10) bereit. Das Bipolar-Maulteilwerkzeug (1) ist mit einem ersten Verbundbau-Maulteil (2), das einen ersten Wirkabschnitt (24) aus einem Leitermaterial und einen ersten Betätigungsabschnitt (20) aus einem Isolatormaterial aufweist, und mit einem zweiten Verbundbau-Maulteil (2'), das einen zweiten Wirkabschnitt (24') aus einem Leitermaterial und einen zweiten Betätigungsabschnitt (20') aus einem Isolatormaterial aufweist ausgestattet, und mit einem Verbundbau-Betätigungselement (3) aus einem Leitermaterial und einem Isolatormaterial, sowie einer Gabelhalterung (4) aus einem Leitermaterial, die zwei Armabschnitte (50) und zumindest einen Rohrabschnitt (60) aufweist, durch den sich das Verbundbau-Betätigungselement (3) zum Eingriff mit den beiden Verbundbau-Maulteilen (2, 2') erstreckt, die um jeweils eine Schwenkachse (8) beweglich zwischen den Armabschnitten (50) gelagert sind. Dabei ist der Wirkabschnitt (24) des ersten Verbundbau-Maulteils (2) mit dem Verbundbau-Betätigungselement (3) elektrisch verbunden und durch den ersten Betätigungsabschnitt (20) von der Gabelhalterung (4) elektrisch isoliert, und der zweite Wirkabschnitt (24') des zweiten Verbundbau-Maulteils (2') ist mit der Gabelhalterung (4) elektrisch verbunden und durch den zweiten Betätigungsabschnitt (20) von dem Verbundbau-Betätigungselement (3) elektrisch isoliert ist. Das Verbundbau-Betätigungselement (3) und die Gabelhalterung (4) sind elektrisch voneinander isoliert.

## Beschreibung

Die Erfindung betrifft ein chirurgisches Bipolar-Maulteilwerkzeug, ein chirurgisches Bipolar-Instrument mit einem Bipolar-Maulteilwerkzeug und ein Verfahren zur Herstellung des chirurgischen Bipolar-Maulteilwerkzeugs.

Aus dem Stand der Technik ist bekannt, chirurgische Instrumente mit einem Maulteilwerkzeug aus beweglichen Maulteilen, wie z. B. Präparier-, Fasszangen und Scheren anzuwenden, die je nach Form und Größe der Maulteile eine Präparier-, Fass- oder Schneidefunktion haben. Das Maulteilwerkzeug ist zur Anordnung an dem distalen Ende eines Außenschafts vorgesehen, der an seinem proximalen Ende mit einer Betätigungseinrichtung (z. B. Griff oder robotische Handhabe) verbunden ist. Zum Öffnen und Schließen der Maulteile weist das Maulteilwerkzeug eine Maulteilmechanik auf, die über ein Betätigungselement, das sich durch den Außenschaft erstreckt, mit der Betätigungseinrichtung verbunden ist. Die Maulteilmechanik umfasst zumindest ein Gelenk, das eine Gelenkachse bereitstellt, um die zumindest eines der beiden Maulteile bewegt werden kann. Man unterscheidet zwischen einseitig öffnenden Maulteilwerkzeugen, bei denen nur ein Maulteil bewegt wird, und beidseitig öffnenden Maulteilwerkzeugen, bei denen beide Maulteile bewegt werden.

Die DE 10 2006 028 001 B4 beschreibt eine chirurgische Greifzange mit zwei um jeweils eine Schwenkachse beweglich an einem gegabelten Grundkörper gelagerten Greifbacken. Der Grundköper weist dazu zwei Gabelarmabschnitte auf, die sich von einem Rohrabschnitt erstrecken, durch den sich ein Betätigungselement zum Eingriff mit den Greifbacken erstreckt. Jede Schwenkachse wird durch einen an der Greifbacke ausgebildeten Schwenkzapfen bereitgestellt, der in einer Ausnehmung am Gabelarmabschnitt des Grundkörpers aufgenommen ist. Ferner hat jede Greifbacke eine Kopplungsöffnung zum Eingriff mit einem an dem Betätigungselement ausgebildeten Antriebszapfen. Zwischen dem Schwenkzapfen und der Kopplungsöffnung befindet sich an jeder Greifbacke eine sichelförmige Führungsausnehmung, durch die ein in beiden Gabelarmabschnitten festsitzender Führungsbolzen geführt ist.

Damit kommt die chirurgische Greifzange ohne seitliche Gelenkteile aus, ist allerdings mit diesem Bauprinzip nur zur stromlosen oder monopolaren Hochfrequenz-Anwendung geeignet. Ein Maulteilwerkzeug mit bipolarer Hochfrequenz-Funktion für die Hochfrequenz-Chirurgie lässt sich mit dem offenbarten Aufbau nicht realisieren.

DE 698 23 862 T2 offenbart ein biopolares elektrochirurgisches Laparoskopieinstrument zum Versiegeln von Gefäßen und vaskulärem Gewebe. Es weist einen Gabelkopf auf, an dem zwei schwenkbare Maulteile angeordnet sind. Diese Maulteile bestehen vollständig aus einem einzelnen Stück Edelstahl oder einem anderen elektrisch leitfähigen Material. Der Gabelkopf besteht aus einem elektrisch isolierenden Material, wodurch die Maulteile elektrisch voneinander isoliert werden.

Ausgehend von diesem Stand der Technik ist es Aufgabe der vorliegenden Erfindung, ein chirurgisches Bipolar-Maulteilwerkzeug bereitzustellen, das ohne seitlich austretende Gelenkteile mit geringer Bauteilanzahl einfach montiert werden kann.

Diese Aufgabe wird durch ein chirurgisches Bipolar-Maulteilwerkzeug mit den Merkmalen des Anspruchs 1 gelöst. Bevorzugte Ausführungsformen sind in den Unteransprüchen ausgeführt.

Die Bereitstellung eines entsprechend modifizierten chirurgischen Bipolar-Instruments wird durch das chirurgische Bipolar-Instrument mit den Merkmalen des Anspruchs 15 gelöst.

Die weitere Aufgabe, eine einfache Montage des chirurgischen Bipolar-Maulteilwerkzeugs bereitzustellen, wird durch das Verfahren mit den Merkmalen des unabhängigen Anspruchs 16 gelöst.

Gemäß einer ersten Ausführungsform weist das chirurgische Bipolar-Maulteilwerkzeug zwei Maulteile, ein Betätigungselement, das eine Längsachse definiert, und eine Gabelhalterung auf. Diese hat zwei Armabschnitte und zumindest einen Rohrabschnitt, durch den sich das Betätigungselement zum Eingriff mit den zwei Maulteilen erstreckt, die um jeweils eine Schwenkachse beweglich zwischen den zwei Armabschnitten gelagert sind. Vorteilhaft sind die Maulteile des chirurgischen Bipolar-Maulteilwerkzeugs in Verbundbauweise aufgebaut, wobei ein ersten Verbundbau-Maulteil einen ersten Wirkabschnitt aus einem Leitermaterial und einen ersten Betätigungsabschnitt aus einem Isolatormaterial aufweist. Ein zweites Verbundbau-Maulteil weist entsprechend einen zweiten Wirkabschnitt aus einem Leitermaterial und einen zweiten Betätigungsabschnitt aus einem Isolatormaterial auf. Auch das Betätigungselement ist in Verbundbauweise als Verbundbau-Betätigungselement aus einem Leitermaterial und einem Isolatormaterial gebildet. Die Gabelhalterung hingegen ist aus einem Leitermaterial gefertigt. Dabei ist der erste Wirkabschnitt des ersten Verbundbau-Maulteils mit dem Verbundbau-Betätigungselement elektrisch verbunden und durch den ersten Betätigungsabschnitt von der Gabelhalterung elektrisch isoliert. Der zweite Wirkabschnitt des zweiten Verbundbau-Maulteils ist mit der Gabelhalterung elektrisch verbunden und durch den zweiten Betätigungsabschnitt von dem Verbundbau-Betätigungselement elektrisch isoliert. Die beiden Stromleitungskomponenten Verbundbau-Betätigungselement und Gabelhalterung sind dabei elektrisch voneinander isoliert.

Durch den Verbundaufbau der Maulteile und des Betätigungselements werden die beiden Stromübertragungswege zu den Wirkabschnitten der Maulteile gegeneinander isoliert und dennoch eine hohe Kraftübertragung ermöglicht.

Leitermaterial meint hier elektrisch leitendes Material, z. B. Metallmaterial mit einer elektrischen Leitfähigkeit größer als 10⁵ S/m, und Isolatormaterial elektrisch isolierendes Material, z. B. Keramik-, Glas- oder Kunststoffmaterial mit einer elektrischen Leitfähigkeit kleiner als 10⁻⁸ S/m. Die Leitermaterialien der Maulteile und des Betätigungselements können sich voneinander unterscheiden oder es können die gleichen Leitermaterialien verwendet werden. Entsprechend können als Isolatormaterialien gleiche oder unterschiedliche Materialien eingesetzt werden. Mit Verbundbau oder Verbundbauweise ist ein Aufbau der Maulteile und des Betätigungselements mit Bauteilen aus verschiedenen Materialien, hier einem Leiter- und einem Isolatormaterial gemeint, die ineinandergreifend, d. h. formschlüssig, und unlösbar miteinander verbunden sind. Das Isolatormaterial kann einen CTI-Wert (Kriechstromfestigkeit) von wenigstens 150, vorzugsweise wenigstens 300, bevorzugt wenigstens 450 und besonders bevorzugt wenigstens 600 aufweisen. Das Isolatormaterial kann ein Kunststoff sein. Beispielsweise kann es sich bei dem Isolationsmaterial um Polyimid, Polyethylen, Polyesterharz, Polytetrafluorethylen, Polybutylenterephthalat, Hexafluorpropylen-Copolymer und / oder Perfluoralkoxy-Polymer handeln. Bevorzugst könnte es sich bei dem Isolationsmaterial um Cycloolefin-Copolymer handeln.

Nach einer weiteren Ausführungsform des chirurgischen Bipolar-Maulteilwerkzeugs weist das erste Verbundbau-Maulteil einen ersten Kopplungsabschnitt aus dem Leitermaterial auf, der mit dem ersten Wirkabschnitt verbunden - vorzugsweise einstückig gefertigt - ist. Dabei sind der erste Kopplungsabschnitt und der erste Betätigungsabschnitt ineinandergreifend verbunden. Entsprechend weist das zweite Verbundbau-Maulteil einen zweiten Kopplungsabschnitt aus dem Leitermaterial auf, der mit dem zweiten Wirkabschnitt verbunden - ebenfalls vorzugsweise einstückig gefertigt - ist. Auch der zweite Kopplungsabschnitt und der zweite Betätigungsabschnitt sind ineinandergreifend verbunden. Beide Maulteile sind damit zweiteilig aus einem Leiterbauteil, das den Wirkabschnitt und Kopplungsabschnitt umfasst, und einem Isolatorbauteil aufgebaut, der den Betätigungsabschnitt bildet, wobei das Leiterbauteil und das Isolatorbauteil formschlüssig und unlösbar über den Kopplungsabschnitt mechanisch miteinander verbunden sind

Ferner weist das Verbundbau-Betätigungselement einen Kopplungseinsatz aus dem Leitermaterial, der mit einem Stangenabschnitt aus dem Leitermaterial verbunden ist, und einen distalen Endabschnitt aus dem Isolatormaterial auf. Der Kopplungseinsatz und der distale Endabschnitt sind ineinandergreifend verbunden, wobei der Stangenabschnitt mit einer Isolatorhülle ummantelt ist, die sich an den distalen Endabschnitt anschließt. So wird die elektrische Verbindung des ersten Wirkabschnitts mit dem Verbundbau-Betätigungselement durch den ersten Kopplungsabschnitt hergestellt, der den Kopplungseinsatz kontaktiert. Und die elektrische Isolierung des Verbundbau-Betätigungselements von der Gabelhalterung wird durch den distalen Endabschnitt und die Isolatorhülle bereitgestellt.

Nach einer weitere Ausführungsform des chirurgischen Bipolar-Maulteilwerkzeugs hat jeder Armabschnitt der Gabelhalterung eine Schwenkausnehmung zur Bildung der jeweiligen Schwenkachse. Und das erste Verbundbau-Maulteil weist an dem ersten Betätigungsabschnitt einen Schwenkzapfen auf, der in der Schwenkausnehmung eines ersten Armabschnitts aufgenommen ist. Dazu hat das erste Verbundbau-Maulteil an dem ersten Kopplungsabschnitt eine Kopplungsöffnung, in der ein erster an dem Kopplungseinsatz ausgebildeter Antriebszapfen des Verbundbau-Betätigungselements aufgenommen ist. Umgekehrt dazu weist zweite Verbundbau-Maulteil an dem zweiten Betätigungsabschnitt eine Kopplungsöffnung auf, in der ein zweiter an dem Kopplungseinsatz ausgebildeter Antriebszapfen des Verbundbau-Betätigungselements aufgenommen ist. Und an dem zweiten Kopplungsabschnitt hat das zweite Verbundbau-Maulteil einen Schwenkzapfen, der in der Schwenkausnehmung eines zweiten Armabschnitts aufgenommen ist.

Nach einer weiteren Ausführungsform des chirurgischen Bipolar-Maulteilwerkzeugs ist der Kopplungseinsatz des Verbundbau-Betätigungselements in einer Formaussparung angeordnet, die in dem distalen Endabschnitt ausgebildet ist. Die Form dieser Formaussparung ist mit dem Kopplungseinsatz abgestimmt. Dabei erstreckt sich der erste Antriebszapfen von einer freien, von dem distalen Endabschnitt abgewandten Oberseite des Kopplungseinsatzes. Und der zweite Antriebszapfen erstreckt sich von einer dem distalen Endabschnitt zugewandten Unterseite des Kopplungseinsatzes durch eine in dem distalen Endabschnitt ausgebildete Zapfenöffnung.

Dabei sind die Antriebszapfen des Verbundbau-Betätigungselements vorzugsweise für einen möglichst großen Hebelarm zur gleichmäßigen Betätigung der Maulteilwerkzeuge von der Längsachse, die durch den Stangenabschnitt definiert wird, in radialer Richtung beabstandet und rotationssymmetrisch ausgebildet.

Gemäß einer noch weiteren Ausführungsform des chirurgischen Bipolar-Maulteilwerkzeugs wird die Verbindung des Kopplungseinsatzes mit dem Stangenabschnitt des Verbundbau-Betätigungselements durch ein Flachprofilstück bereitgestellt, das aus dem Leitermaterial gefertigt ist. Dabei ist ein distales Ende des Flachprofilstücks in einer Aussparung, die in dem Kopplungseinsatz ausgebildet ist, ineinandergreifend aufgenommen. Und ein proximales Ende des Flachprofilstücks ist in einem an dem distalen Ende des Stangenabschnitts ausgebildeten Aufnahmespalt aufgenommen.

Das Ineinandergreifen bzw. der Formschluss des distalen Endes des Flachprofilstücks mit dem Kopplungseinsatz sorgt in Richtung der Längsachse für eine zugfeste Verbindung. Ferner kann der Stangenabschnitt des Verbund-Betätigungselements in einer weiteren Ausführungsform ein von einer Kreisform abweichendes Querschnittsprofil aufweisen, das zwischen den Armabschnitten, die an ihrer nach innen weisenden Seite ein entsprechendes von der Kreisform abweichendes Teilprofil bereitstellen, verdrehsicher geführt wird.

Ferner weist nach noch einer weiteren Ausführungsform des chirurgischen Bipolar-Maulteilwerkzeugs der Kopplungseinsatz an seiner Unterseite zumindest einen Boden-Abstandshalter auf, durch den ein Zwischenraum zwischen der Unterseite des Kopplungseinsatzes und einem Boden der Formaussparung im Endabschnitt gebildet wird. Eine mit dem Zwischenraum verbundene Füllbohrung ist vorzugsweise in dem Kopplungseinsatz oder alternativ ggf. in dem distalen Endabschnitt vorgesehen, sodass der Zwischenraum durch die Füllbohrung mit einem Klebstoff gefüllt wird. Für eine stabile Anordnung des Kopplungseinsatzes in der Formaussparung vor der Befüllung mit Klebstoff können mehrere Boden-Abstandshalter vorgesehen sein. Ein Boden-Abstandshalter kann einen kreis- oder ringförmigen Querschnitt haben, oder auch oval, amorph oder polygonal, eckig oder leisten- bzw. strebenartig geformt sein.

Dabei kann der Kopplungseinsatz nach einer weiteren Ausführungsform des chirurgischen Bipolar-Maulteilwerkzeugs an seiner umfänglichen Kontur zumindest zwei Umfangs-Abstandshalter aufweisen. Durch die Umfangs-Abstandshalter wird der Kopplungseinsatz in der Formaussparung in Bezug zu deren Seitenwänden ausgerichtet, und jeweils eine Fuge wird zwischen der umfänglichen Kontur des Kopplungseinsatzes und den Seitenwänden der Formaussparung ausgebildet. Die Fugen sind mit einem Klebstoff gefüllt.

Vorzugsweise sind dabei die Fugen mit dem Zwischenraum zwischen der Unterseite des Kopplungseinsatzes und dem Boden der Formaussparung verbunden, sodass während der Montage beim Einfüllen des Klebstoffs durch die Füllbohrung in den Zwischenraum Luft durch die Fugen entweichen kann, bis der Klebstoff auch in die Fugen dringt. Mit Füllung der Fugen ist dann sichergestellt, dass der Zwischenraum gleichmäßig und komplett mit dem Klebstoff gefüllt ist.

Analog zur Anordnung des Kopplungseinsatzes in der Formaussparung des distalen Endabschnitts ist nach einer weiteren Ausführungsform des Bipolar-Maulteilwerkzeugs der erste Kopplungsabschnitt in einer Formaussparung angeordnet, die in dem ersten Betätigungsabschnitt ausgebildet ist und eine mit dem ersten Kopplungsabschnitt abgestimmte Form aufweist. Und der zweite Kopplungsabschnitt ist in einer Formaussparung angeordnet, die in dem zweiten Betätigungsabschnitt ausgebildet ist und eine mit dem zweiten Kopplungsabschnitt abgestimmte Form aufweist.

Und zur Fixierung der Kopplungsabschnitte in der jeweiligen Formaussparung mittels Klebstoffs weist jeder Kopplungsabschnitt nach einer noch weiteren Ausführungsform des Bipolar-Maulteilwerkzeugs an seiner Unterseite zumindest einen Boden-Abstandshalter auf, durch den ein Zwischenraum zwischen der Unterseite des Kopplungsabschnitts und einem Boden der Formaussparung ausgebildet wird. Dabei weist jeder Kopplungsabschnitt oder ggf. Betätigungsabschnitt eine Füllbohrung auf, die mit dem Zwischenraum verbunden ist, der mit dem Klebstoff gefüllt wird. Alternativ oder vorzugsweise zusätzlich dazu weist jeder Kopplungsabschnitt an seiner umfänglichen Kontur zumindest zwei Umfangs-Abstandshalter auf, durch die der Kopplungsabschnitt in der Formaussparung in Bezug zu deren Seitenwänden ausgerichtet wird. Dadurch wird jeweils zwischen der umfänglichen Kontur des Kopplungsabschnitts und den Seitenwänden der Formaussparung eine Fuge ausgebildet, die, bevorzugt mit dem Zwischenraum verbunden, mit dem Klebstoff gefüllt ist.

Jedes Verbundbau-Maulteil des Bipolar-Maulteilwerkzeugs hat nach einer weiteren Ausführungsform einen Führungszapfen, und jeder Armabschnitt weist eine zu der Schwenkausnehmung konzentrische Führungsbahn auf. Diese ist zur Führung des Führungszapfens vorgesehen, dessen Abstand von dem Schwenkzapfen einem Abstand der Führungsbahn von der Schwenkausnehmung entspricht.

Ferner wird nach einer weiteren Ausführungsform des Bipolar-Maulteilwerkzeugs die Gabelhalterung aus zwei Gabelarmen, die die Armabschnitte aufweisen, und einem Gabelkörper, der den Rohrabschnitt aufweist, in einer Fügeanordnung gebildet. Dazu weist jeder Gabelarm einen Fügeabschnitt auf und der Gabelkörper hat zwei Anlageabschnitte, die in der Fügeanordnung mit den Fügeabschnitten der Gabelarme verbunden sind. Dabei werden die Fügeabschnitte der Gabelarme mit den Anlageabschnitten des Gabelkörpers in der Fügeanordnung zur Bildung der Gabelhalterung durch eine Formschlussverbindung, eine Stoffschlussverbindung, eine Kraftschlussverbindung oder eine Kombination davon verbunden.

Auf diese Weise wird die Montage des chirurgischen Bipolar-Maulteilwerkzeugs durch das Fügen der Gabelarme mit dem Gabelkörper zur Gabelhalterung ermöglicht. Das so gebildete chirurgische Bipolar-Maulteilwerkzeug kommt dadurch bei vergleichbarer Funktionalität ohne einen zusätzlichen Führungsbolzen aus. Außerdem sind keine Führungsausnehmungen in den Verbundbau-Maulteilen erforderlich, da die in dem Armabschnitt der Gabelhalterung ausgebildete Führungsbahn für den Anschlag zur Begrenzung des Öffnungsraums der Maulteile sorgt.

Zur Formschlussverbindung weisen der Fügeabschnitt und der Anlageabschnitt ineinandergreifende Formgebung auf. Dazu ist an jedem Fügeabschnitt zumindest ein Formelement und an jedem Anlageabschnitt zumindest ein Gegenformelement komplementär zu dem Formelement ausgebildet. Die Stoffschlussverbindung ist eine Schweiß-, Löt- oder Klebeverbindung. Die Kraftschlussverbindung weist zumindest ein Verbindungselement auf, das den Fügeabschnitt an dem Anlageabschnitt hält und aus einer Gruppe ausgewählt ist, die zumindest Niete, Schrauben, Hülsen, Federklammern und Halteklipse umfasst.

Beispiele für Kombinationen der Verbindungsarten umfassen eine Formschlussverbindung mit einer Stoffschlussverbindung, eine Formschlussverbindung mit einer Kraftschlussverbindung und eine Kraftschlussverbindung mit einer Stoffschlussverbindung. Bevorzugte Verbindungsarten sind Formschlussverbindungen und Stoffschlussverbindungen, da sie ohne zusätzliche Verbindungselemente auskommen.

Die Formschlussverbindung wirkt durch das Formschluss-Paar aus Formelement und Gegenformelement als ineinandergreifende Verbindungspartner, die einander kontaktierende Wirkflächen aufweisen, an denen Normalkräfte übertragen werden. In dieser Richtung blockiert jeder Verbindungspartner die Bewegung des anderen. In Fügerichtung der Verbindungspartner, d. h. parallel zu den Wirkflächen, sind Formschlussverbindungen im Allgemeinen lösbar. Bei einer Stoffschlussverbindung verhindern Kohäsions- und Adhäsionskräfte jegliche Bewegung zwischen den Verbindungspartnern, die somit nicht lösbar verbunden sind. Eine Kraftschlussverbindung auch Reibschlussverbindung genannt, verhindert die Bewegung der Verbindungspartnern durch ein mechanisches Verbindungselement, das eine Normalkraft auf die Verbindungsflächen bereitstellt und die Verbindungspartner durch Haftreibung zusammenhält.

Vorteilhaft kann in einer bevorzugten Ausführungsform des chirurgischen Bipolar-Maulteilwerkzeugs eine Formschlussverbindung auch zur Ausrichtung der Armabschnitte parallel zu der Längsachse genutzt werden. Ein ungewolltes Lösen der Formschlussverbindung in Fügerichtung kann durch die Kombination mit einer Stoff- oder Kraftschlussverbindung verhindert werden.

Ferner kann gemäß einer weiteren Ausführungsform des chirurgischen Bipolar-Maulteilwerkzeugs vorgesehen sein, dass der Gabelkörper proximalseitig an dem Rohrabschnitt einen abgesetzten, im Durchmesser reduzierten Kupplungsabschnitt und/oder zumindest ein Kupplungselement aufweist, das zur Verbindung mit einem Außenschaft ausgebildet ist, in dem das Betätigungselement längsbeweglich anordenbar ist.

Eine Verbindung des Gabelkörpers, bzw. der Gabelhalterung, mit einem Außenschaft kann dann beispielsweise die Aufnahme des Kupplungsabschnitts in einem distalen End- bzw. Kupplungsabschnitt des Außenschafts unter dessen Anlage an dem Absatz zum Rohrabschnitt umfassen. Dabei ist der reduzierte (Außen-)Durchmesser des Kupplungsabschnitts an den Innendurchmesser des Außenschaft-Kupplungsabschnitts angepasst.

Alternativ oder zusätzlich können die Kupplungselemente zur Verbindung des Gabelkörpers mit dem Außenschaft genutzt werden, der dazu entsprechende Kuppelelemente zum Eingriff mit den Kupplungselementen aufweisen kann. Beispielhafte Kupplungselemente sind ein Gewinde oder Bajonett-Nocken, die mit einem Gegengewinde oder einer Bajonett-Nut als Kuppelelemente in lösbaren Eingriff bringbar sind. Üblicherweise befindet sich an dem proximalen Ende des Außenschafts eine manuelle oder robotische Betätigungseinrichtung, die mit dem distalen chirurgischen Bipolar-Maulteilwerkzeug ein chirurgisches Bipolar-Instrument bildet. Das Verbundbau-Betätigungselement ist dabei mit der Betätigungseinrichtung zur Betätigung der Verbundbau-Maulteile verbunden.

Ferner kann das chirurgische Bipolar-Maulteilwerkzeug nach einer weiteren Ausführungsform eine Haltehülse als Haltelement mit einem distalen und einem proximalen Halteabschnitt aufweisen. Der distale Halteabschnitt umgibt den Rohrabschnitt in der Fügeanordung, und der proximale Halteabschnitt ist dazu ausgebildet, in einer Verbindungsanordnung des Gabelkörpers mit dem Außenschaft dessen distalen Kupplungsabschnitt mit dem darin angeordneten Kupplungsabschnitt aufzunehmen. Der distale Halteabschnitt der Haltehülse ist dazu an den Rohrabschnitt des Gabelkörpers und der proximale Halteabschnitt an dem distalen Kupplungsabschnitt des Außenschafts ausgebildet. D. h, dass eine Länge und ein Innendurchmesser des distalen Halteabschnitts der Haltehülse an die Länge und den Außendurchmesser des Rohrabschnitts und eine Länge und ein Innendurchmesser des proximalen Halteabschnitts an die Länge und den Außendurchmesser des distalen Kupplungsabschnitts des Außenschafts angepasst sind. Dabei ist der proximale Halteabschnitt von dem distale Halteabschnitt der Haltehülse radial abgesetzt, d. h. mit reduzierten Außendurchmesser ausgebildet.

Nach einer noch weiteren Ausführungsform kann das chirurgische Bipolar-Maulteilwerkzeug eine Verbindungshülse aufweisen, die die Verbindung der der Gabelhalterung mit dem Außenschaft sichert. Die Verbindungshülse weist dazu an einem proximalen Verbindungsabschnitt ein Verbindungselement auf, das zur Verbindung mit einem proximalseitig des Kupplungselements angeordneten Verbindungselement des Außenschafts ausgebildet ist. Distalseitig schlägt die Verbindungshülse an einem radialen Absatz der Gabelarme an, der zwischen dem Armabschnitt und dem Verbindungsabschnitt ausgebildet ist. Dann schießt die zylindrische Außenoberfläche der Verbindungshülse bündig an die teilzylindrische Außenfläche der Armabschnitte des Rohrabschnitts an. Der proximale Verbindungsabschnitt ist dabei radial von dem distalen Anschlagsabschnitt der Verbindungshülse abgesetzt, d. h. mit reduzierten Außendurchmesser ausgebildet.

Eine beispielhafte Ausführungsform bezieht sich darauf, dass das chirurgische Bipolar-Maulteilwerkzeug sowohl eine Haltehülse, die die Fügeanordung kraftschlüssig zusammenhält und die Kupplungsanordnung des Gabelkörpers mit dem Außenschaft umgibt, als auch eine Verbindungshülse aufweisen kann, die zur Sicherung der Verbindung der Gabelhalterung mit dem Außenschaft die Haltehülse umgibt und beidseitig darüber hinausragt. Dabei ist eine Innenkontur der Verbindungshülse an die Außenkontur der Haltehülse angepasst, wobei die Innenkontur der Verbindungshülse im distalen Anschlagsabschnitt mit einem radialen Innenabsatz ausgebildet ist, an dem die Haltehülse mit dem Absatz zwischen dem proximalen und distalen Halteabschnitt zur Anlage kommt.

Nach einer weiteren Ausführungsform des chirurgischen Bipolar-Maulteilwerkzeugs liegen die Anlageabschnitte diametral an dem Rohrabschnitt des Gabelkörpers vor. Ferner sind die Anlageabschnitte des Gabelkörpers und die Fügeabschnitte der Gabelarme zur Ausrichtung der Armabschnitte parallel zu einer Rotationsachse des Rohrabschnitts ausgebildet. Dadurch erstrecken sich die Armabschnitte in der Fügeanordnung von dem Rohrabschnitt diametral und parallel in Bezug auf die Längsachse.

Nach noch einer weiteren Ausführungsform des chirurgischen Bipolar-Maulteilwerkzeugs ist vorgesehen, dass jeder Anlageabschnitt an dem Rohrabschnitt des Gabelkörpers durch eine Ausnehmung gebildet ist, die sich zu dessen distalen Ende erstreckt. Der Fügeabschnitt jedes Gabelarms ist komplementär zu dieser Ausnehmung ausgebildet, wobei der Fügeabschnitt in der Fügeanordnung an dem Anlageabschnitt eine Zylinderform des Rohrabschnitts bündig ergänzt. Dazu können die Ausnehmung an dem Rohrabschnitt zur Bildung des Anlageabschnitts und der die Ausnehmung ergänzende Fügeabschnitt bevorzugt zylindersegmentförmig ausgebildet sein. Dadurch haben die Fügeabschnitte und die Anlageabschnitte ebene Kontaktflächen, die sich parallel zur Längsachse erstrecken.

Gemäß einer Weiterbildung des chirurgischen Bipolar-Maulteilwerkzeugs, dessen Gabelhalterung durch eine Formschlussverbindung erhalten wird, können an jedem Fügeabschnitt zwei oder mehr Formelemente ausgebildet sein. Entsprechend sind dann an jedem Anlageabschnitt zwei oder mehr Gegenformelemente ausgebildet, die komplementär zu dem zwei oder mehr Formelementen des Fügeabschnitts ausgebildet sind. Zusätzlich oder alternativ dazu kann gemäß einer weiteren Ausführungsform des chirurgischen Bipolar-Maulteilwerkzeugs das Formelement an dem Fügeabschnitt ein Langloch und das Gegenformelement an dem Anlageabschnitte ein Passfederprofil sein.

Unter Langloch wird eine längliche Durchgangsöffnung oder Nut verstanden, deren Längsseiten parallel zueinander verlaufen. Die schmalen Langlochseiten sind vorzugsweise abgerundet, wobei ein Rundungsdurchmesser (Kreisdurchmesser oder elliptische Haupt- oder Nebenachse) der Breite des Langlochs entspricht. Alternativ ist es aber auch möglich, dass die schmalen Langlochseiten geradlinig sein können, sodass das Langloch Rechteck- oder Trapezform hat. Denkbar ist ferner, dass die schmalen Langlochseiten eckig geformt sind, sodass das Langloch eine Sechs- oder Mehreckform mit konkaven oder konvexen Schmalseiten hat. Das Passfederelement ist entsprechend zur formschlüssigen Aufnahme in dem Langloch ausgebildet und weist daher einen länglichen Körper mit parallelen Längsseiten auf. Dabei kann das Passfederelement eine Quaderform oder eine quaderähnliche Form aufweisen, deren Schmalseiten entsprechend den Langloch-Schmalseiten abgerundet, gerade oder eckig verlaufen. Jedes Formschluss-Paar aus Langloch und Passfederelement kann an den jeweiligen Füge- und Anlageabschnitten mit den Längsseiten beispielsweise parallel oder quer zur Längsachse ausgebildet sein. Sind an jedem Füge- und Anlageabschnitt jeweils zwei oder mehr Formschluss-Paare aus Langloch und Passfederelement vorgesehen, kann ein Formschluss-Paar parallel zur Längsachse und ein anderes quer zur Längsachse orientiert sein.

Nach einer weiteren Ausführungsform des chirurgischen Bipolar-Maulteilwerkzeugs sind die Formelemente an den Fügeabschnitten der zwei Gabelarme gleich ausgebildet und die Anlageabschnitte mit den Gegenformelementen des Gabelkörpers rotationssymmetrisch ausgebildet. Da die Fügeabschnitte der Gabelarme die gleichen Formelemente aufweisen, können die Gabelarme bei der Montage beliebig an den bezüglich der Längsachse rotationssymmetrischen Anlageabschnitten gefügt werden.

Ein ebenfalls chirurgisches Bipolar-Instrument weist ein chirurgisches Maulteilwerkzeug mit zwei Maulteilen, einem Betätigungselement und einer Gabelhalterung auf. Das Betätigungselement erstreckt sich zum Eingriff mit den zwei Maulteilen durch die Gabelhalterung, in der die beiden Maulteile schwenkbar um zumindest eine Schwenkachse gelagert sind. Die Gabelhalterung ist an einem distalen Ende eines Außenschafts angeordnet, durch den sich das Betätigungselement längsbeweglich erstreckt, das mit einer Betätigungseinrichtung in Eingriff steht, die an einem proximalen Ende des Außenschafts angeordnet ist. Erfindungsgemäß ist das chirurgische Maulteilwerkzeug ein chirurgisches Bipolar-Maulteilwerkzeug nach einer der vorbeschriebenen erfindungsgemäßen Ausführungsformen.

Ein Verfahren zur Herstellung eines chirurgischen Bipolar-Maulteilwerkzeugs umfasst gemäß einer ersten Ausführungsform zunächst in einem Schritt S0) das Bereitstellen eines Montagesets, das die zwei Verbundbau-Maulteile, das Verbundbau-Betätigungselement und eine Gabelhalterung aus einem Leitermaterial umfasst. Dazu gehört das Herstellen der Verbundbau-Maulteile und des Verbundbau-Betätigungselements durch
- Fertigen eines ersten Wirkabschnitts aus einem Leitermaterial und Fertigen eines ersten Betätigungsabschnitts aus einem Isolatormaterial und Verbinden des ersten Wirkabschnitts und des ersten Betätigungsabschnitts zu dem ersten Verbundbau-Maulteil, und
- Fertigen eines zweiten Wirkabschnitts aus einem Leitermaterial und Fertigen eines zweiten Betätigungsabschnitts aus einem Isolatormaterial und Verbinden des zweiten Wirkabschnitts und des zweiten Betätigungsabschnitts zu dem zweiten Verbundbau-Maulteil, und
- Fertigen des Verbundbau-Betätigungselement aus einem Leitermaterial und einem Isolatormaterial. Bei der folgenden Montage in Schritt S1) werden die beiden Verbundbau-Maulteile mit dem Verbundbau-Betätigungselement in Eingriff gebracht und in der Gabelhalterung gelagert, wobei
- elektrisches Verbinden des ersten Wirkabschnitts des ersten Verbundbau-Maulteils mit dem Verbundbau-Betätigungselement und elektrisches Isolieren des ersten Wirkabschnitts von der Gabelhalterung durch den ersten Betätigungsabschnitt, und
- elektrisches Verbinden des zweiten Wirkabschnitts des zweiten Verbundbau-Maulteils mit der Gabelhalterung und elektrisches Isolieren des zweiten Wirkabschnitts von dem Verbundbau-Betätigungselement durch den zweiten Betätigungsabschnitt, und
- elektrisches Isolieren des Verbundbau-Betätigungselements und der Gabelhalterung voneinander.

Nach einer weiteren Ausführungsform des erfindungsgemäßen Verfahrens, bei dem in Schritt S0 eine mehrteilige Gabelhalterung bereitgestellt wird, die aus zwei Gabelarmen und einem Gabelkörper besteht, umfasst die Montage in Schritt S1 die folgenden Schritte
S2) Lagern des ersten Verbundbau-Maulteils an einem ersten der zwei Gabelarme, dabei Aufnehmen des Schwenkzapfens in der Schwenkausnehmung und Aufnehmen des Führungszapfens in der Führungsbahn;
S3) in Eingriff Bringen des in den Gabelkörper eingeführten Verbundbau-Betätigungselements mit dem ersten Verbundbau-Maulteil durch Aufnehmen eines ersten an dem distalen Endabschnitt ausgebildeten Antriebszapfens in der Kopplungsöffnung des ersten Verbundbau-Maulteils, und Anordnen des ersten Gabelarms mit dem Fügeabschnitt an einem ersten der zwei Anlageabschnitte des Gabelkörpers;
S4) in Eingriff Bringen des zweiten Verbundbau-Maulteils mit dem Verbundbau-Betätigungselement durch Aufnehmen eines zweiten an dem Endabschnitt ausgebildeten Antriebszapfens in der Kopplungsöffnung des zweiten Verbundbau-Maulteils; und
S5) Anordnen des zweiten der zwei Gabelarme unter Lagerung des zweiten Verbundbau-Maulteils durch Aufnehmen des Schwenkzapfens in der Schwenkausnehmung und Aufnehmen des Führungszapfens in der Führungsbahn, und
   Anordnen des zweiten Gabelarms mit dem Fügeabschnitt an einem zweiten der zwei Anlageabschnitte des Gabelkörpers;
S6) Bilden der Gabelhalterung aus den zwei Gabelarmen und dem Gabelkörper in der Fügeanordnung durch Verbinden des ersten Fügeabschnitts mit dem ersten Anlageabschnitt und des zweiten Fügeabschnitts mit dem zweiten Anlageabschnitt, und Erhalten des chirurgischen Bipolar-Maulteilwerkzeugs.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- Fig. 1: eine perspektivische Ansicht eines chirurgischen Bipolar-Maulteilwerkzeugs nach einer erfindungsgemäßen Ausführungsform,
- Fig. 2: eine weitere perspektivische Ansicht des chirurgischen Bipolar-Maulteilwerkzeugs aus Fig. 1 mit geöffneten Maulteilen,
- Fig. 3: eine Seitenansicht eines chirurgischen Bipolar-Instruments mit einem Maulteilwerkzeug nach einer erfindungsgemäßen Ausführungsform,
- Fig. 4: eine perspektivische Ansicht eines Montagesets für das chirurgische Bipolar-Maulteilwerkzeug aus Fig. 1,
- Fig. 5: eine perspektivische Teilexplosionsansicht des distalen Endabschnitts und Kopplungseinsatzes eines Betätigungselements des Montagesets aus Fig. 4,
- Fig. 6: eine perspektivische Ansicht des mit dem Stangenabschnitt gefügten distalen Endabschnitts aus Fig. 5,
- Fig. 7: eine perspektivische Teilexplosions-Rückansicht mit transparent dargestelltem distalen Endabschnitt aus Fig. 6,
- Fig. 8: eine Seitenansicht auf das distale Ende des Betätigungselements aus Fig. 4,
- Fig. 9: eine perspektivische Ansicht eines ersten Maulteils des Montagesets aus Fig. 4,
- Fig. 10: eine weitere perspektivische Ansicht des ersten Maulteils aus Fig. 9,
- Fig. 11: eine perspektivische Explosionsansicht des ersten Maulteils aus Fig. 9 mit transparent dargestelltem Betätigungsabschnitt,
- Fig. 12: eine perspektivische Seitenansicht des zweiten Maulteils des Montagesets aus Fig. 4,
- Fig. 13: eine weitere perspektivische Seitenansicht des zweiten Maulteils aus Fig. 12,
- Fig. 14: eine perspektivische Innenansicht eines Gabelarms des Montagesets aus Fig. 4,
- Fig. 15: eine perspektivische Seitenansicht eines Gabelkörpers des Montagesets aus Fig. 4,
- Fig. 16: eine perspektivische Ansicht des chirurgischen Bipolar-Maulteilwerkzeugs aus Fig. 1 in einer Verbindungsanordnung mit einem Außenschaft eines chirurgischen Bipolar-Instruments,
- Fig. 17: eine perspektivische Ansicht entsprechend Fig. 16 in einer Ausführungsform mit Sicherungshülse,
- Fig. 18: eine perspektivische Ansicht entsprechend Fig. 17 in einer Ausführungsform mit Verbindungshülse.

Die Erfindung betrifft ein chirurgisches Bipolar-Maulteilwerkzeug und ein chirurgisches Bipolar-Instrument, das mit einem solchen chirurgischen Bipolar-Maulteilwerkzeug ausgestattet ist. Außerdem betrifft die Erfindung ein Verfahren zur Herstellung des chirurgischen Bipolar-Maulteilwerkzeugs. Die Gelenkmechanik des chirurgischen Bipolar-Maulteilwerkzeugs eignet sich für Zangensysteme zum Präparieren, Fassen oder Schneiden. Dabei kommt die Gelenkmechanik ohne Gelenkteile aus, die seitlich bzw. in radialer Richtung über einen Durchmesser hinausragen, der durch die Gabelhalterung des Bipolar-Maulteilwerkzeugs bzw. durch den Außenschaft des chirurgischen Bipolar-Instruments definiert wird.

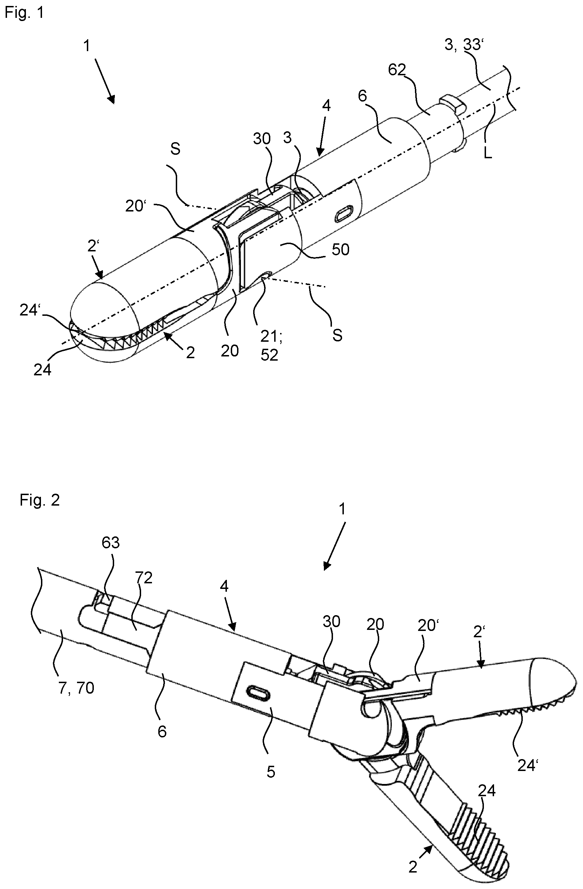

Fig. 1 und 2 zeigen ein Beispiel für ein erfindungsgemäßes chirurgisches Bipolar-Maulteilwerkzeug 1, dessen zwei Verbundbau-Maulteile 2, 2' jeweils um eine Schwenkachse S beweglich zwischen zwei Armabschnitten 50 einer Gabelhalterung 4 gelagert sind. Dabei steht ein Verbundbau-Betätigungselement 3 mit den Verbundbau-Maulteilen 2, 2' des Bipolar-Maulteilwerkzeugs 1 in Eingriff, sodass eine Betätigung an der Verbundbau-Betätigungseinrichtung 3 ein Drehmoment um die Schwenkachse S zum Öffnen oder Schließen der Verbundbau-Maulteile 2, 2' bewirkt. Verbundbau meint hier einen ineinandergreifenden Aufbau aus einem Leiterbauteil, d. h. einem Bauteil aus einem elektrisch leitenden Material, z. B. Metall, und aus einem Isolatorbauteil, d. h. einem Bauteil aus einem elektrisch isolierenden Material, z. B. Keramik oder Kunststoff. Durch den Verbundaufbau der Verbundbau-Maulteile 2, 2' und des Verbundbau-Betätigungselements 3 können in dem Bipolar-Maulteilwerkzeug 1 die beiden Stromübertragungswege für die Pole des bipolaren Hochfrequenz-Stroms gegeneinander isoliert werden.

Das beispielhaft gezeigte chirurgische Bipolar-Maulteilwerkzeug 1 ist eine Fasszange, deren Verbundbau-Maulteile 2, 2'als Elektroden Wirkabschnitte 24, 24' aus elektrisch leitendem Material aufweisen. Im vorliegenden Beispiel sind die Wirkabschnitte 24, 24' ferner zum Fassen von Gegenständen bzw. von Gewebe ausgebildet. In nicht abgebildeten Modifikationen des chirurgischen Bipolar-Maulteilwerkzeugs 1 können die Wirkabschnitte 24, 24' in anderer Weise zum Fassen, oder zum Präparieren oder Schneiden ausgebildet sein.

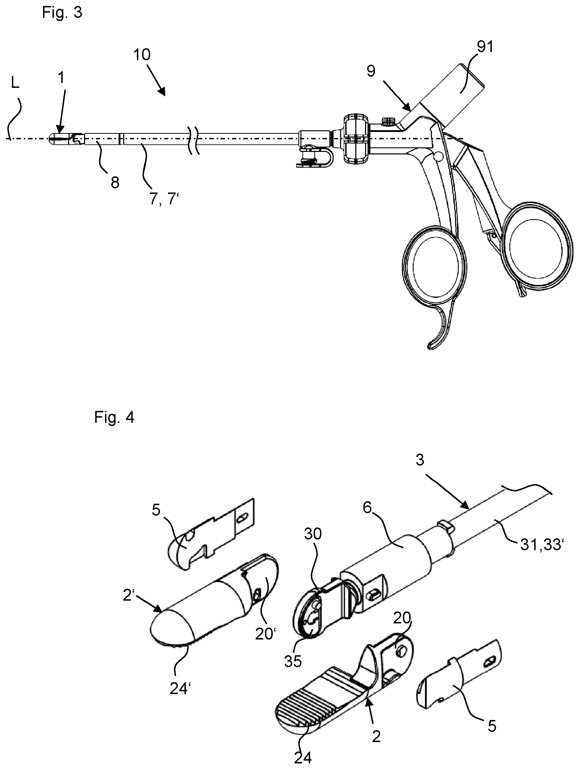

Das Bipolar-Maulteilwerkzeug 1 ist an dem distalen Ende eines Außenschafts 7 angeordnet, der dazu einen distalen Kupplungsabschnitt 70 aufweist, der in Fig. 2 zu sehen ist. Das in Fig. 3 vereinfacht dargestellte erfindungsgemäße chirurgische Bipolar-Instrument 10 hat ferner eine Betätigungseinrichtung 3, hier ein Handgriff 9, am proximalen Ende des Außenschafts 7. Die Betätigungseinrichtung 3 steht mit dem Verbundbau-Betätigungselement 3 in Eingriff, das eine Längsachse L definiert und sich längsbeweglich durch den Außenschaft 7 bis in die Gabelhalterung 4 erstreckt. Zur Vereinfachung werden nachfolgend die Verbundbau-Maulteile 2, 2' und das Verbundbau-Betätigungselement 3 abgekürzt als Maulteile 2, 2' und Betätigungselement 3 bezeichnet.

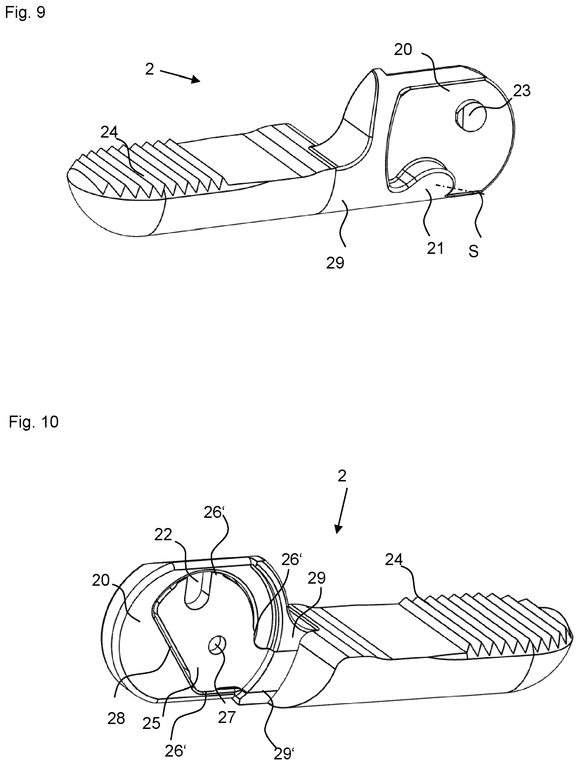

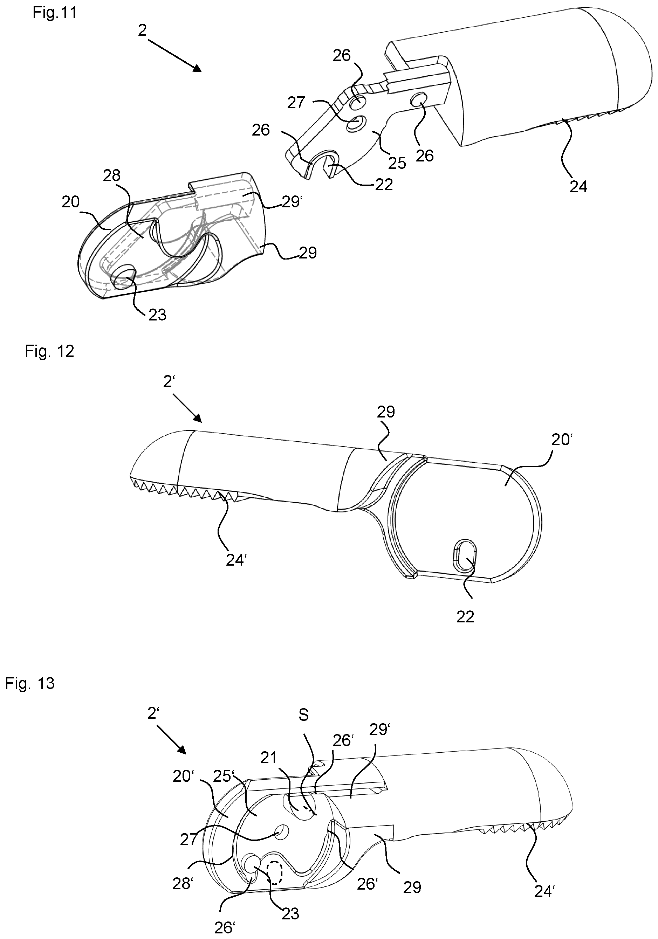

Eine erste Elektrode des chirurgischen Bipolar-Maulteilwerkzeugs 1 wird durch den Wirkabschnitt 24 eines ersten Maulteils 2 bereitgestellt, das im Detail in Fig. 9 bis 11 abgebildet ist. Der Wirkabschnitt 24 des ersten Maulteils 2 wird elektrisch leitend mit dem Betätigungselement 3 verbunden. Die zweite Elektrode wird durch den Wirkabschnitt 24' des zweiten Maulteils 2' gebildet, das in Fig. 12 und 13 dargestellt ist. Der Wirkabschnitt 24' des zweiten Maulteils 2' wird über die Gabelhalterung 4 elektrisch leitend mit dem Außenschaft 7 verbunden. Das erste Maulteil 2 ist daher durch seinen Verbundaufbau elektrisch gegenüber der Gabelhalterung 4 isoliert, und das zweite Maulteil 2' durch seinen Verbundaufbau gegenüber dem Betätigungselement 3, das wiederum gegenüber der Gabelhalterung 4 und dem Außenschaft 7 elektrisch isoliert ist.

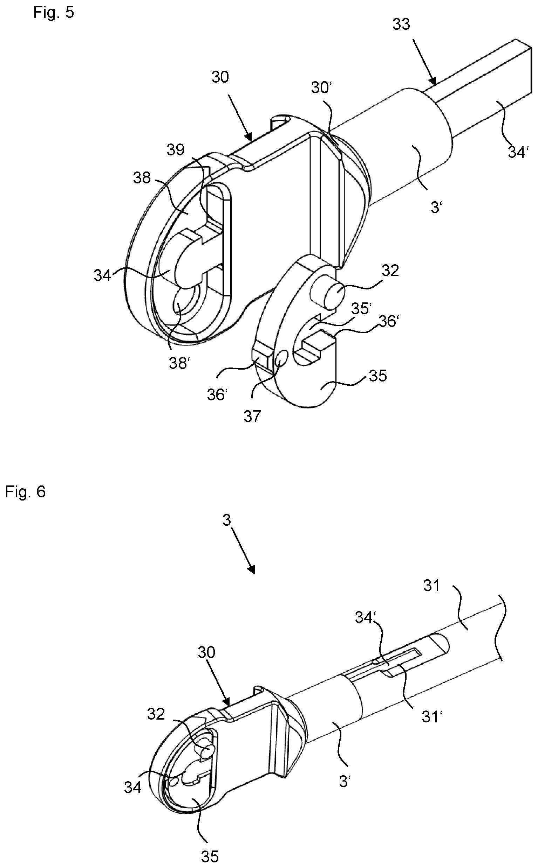

Im Folgenden wird der Aufbau des chirurgischen Bipolar-Maulteilwerkzeugs 1 anhand des Montageablaufs beschrieben, wobei zuerst die Fertigung des Betätigungselements 3 mit seinem distalen Endabschnitt 30 (Fig. 5 bis 8) als Verbundbauteil beschrieben wird. Fig. 5 zeigt den distalen Endabschnitt 30 des Betätigungselements 3, der proximalseitig einen zylindrischen Ansatz 3' aufweist und aus einem elektrisch isolierenden Material, vorzugsweise Keramik, gefertigt ist. Alternativ einsetzbare elektrisch isolierende Materialien finden sich beispielsweise unter den Gläsern und Kunststoffen. Abgesehen von dem zylindrischen Ansatz 3' ist der Endabschnitt 30 laschenartig, d. h. flächig mit parallelen Seitenflächen und abgerundetem Ende, geformt. Die parallelen Seitenflächen des Endabschnitts 30 sind zu dem zylindrischen Ansatz 3' hin durch einen keilartigen Abschnitt 30' begrenzt. An einer Seitenfläche weist der laschenartige Endabschnitt 30 eine annähernd halbkreisförmige Formaussparung 38 auf, die mit einer Zapfenöffnung 38' einen Durchbruch zu der anderen Seitenfläche des laschenartigen Endabschnitts 30 aufweist.

Ferner ist die Formaussparung 38 mit einer sich durch den zylindrischen Ansatz 3' erstreckenden Durchgangsöffnung 39 verbunden, durch die von proximaler Seite ein Flachprofilstück 33 aus einem elektrisch leitenden Material, vorzugsweise Metall, eingeschoben wird. Als Flachprofilstück 33 kann z. B. ein lasergeschnittener Blechstreifen eingesetzt werden. Für eine definierte Führung in Bezug auf die Längsachse L entspricht das Querschnittprofil der Durchgangsöffnung 39 dem des Flachprofilstücks 33. Das distale Ende 34 des Flachprofilstücks 33, das dann in die Formaussparung 38 ragt, ist als eine hier doppelseitige Hakenstruktur geformt. In die Formaussparung 38 des Endabschnitts 30 aus elektrisch isolierendem Material wird ein scheibenartiger Kopplungseinsatz 35 aus elektrisch leitendem Material, vorzugsweise Metall, eingesetzt. Die Form des scheibenartigen Kopplungseinsatzes 35 und der Formaussparung sind aufeinander abgestimmt, sodass der Kopplungseinsatz 35 hier annähernd halbkreisförmig ausgebildet ist. Der Kopplungseinsatz 35 weist ferner eine komplementär zum distalen Ende 34 des Flachprofilstücks 33 geformte Hakenaussparung 35' auf. Diese kommt bei Anordnung des Kopplungseinsatzes 35 in der Formaussparung 38 mit dem distalen Ende 34 des Flachprofilstücks 33 in Eingriff. Der Kopplungseinsatz 35 fixiert das Flachprofilstück 33 in Bezug auf den Endabschnitt 30 und bildet damit eine formschlüssige Verbindung in Richtung der Längsachse L. Dabei schließt der Kopplungseinsatz 35 mit seiner aus der Formaussparung 38 weg weisenden freien Oberseite bündig und eben mit dem distalen Ende 34 des Flachprofilstücks 33 ab und erstreckt sich im Wesentlichen in der Ebene der Seitenfläche des Endabschnitts 30. Vorzugsweise ragt der Kopplungseinsatz 35 mit seiner Oberseite etwas über die Seitenfläche des Endabschnitts 30 hinaus, um einen guten elektrischen Kontakt mit dem ersten Maulteil 2 sicherzustellen.

In Fig. 6 ist der elektrisch isolierende Endabschnitt 30 in gefügtem Zustand mit dem elektrisch leitenden Kopplungseinsatz 35 und dem elektrisch leitenden Flachprofilstück 33 dargestellt. Das Flachprofilstück 33 ist an seinem proximalen Ende 34' mit einem Stangenabschnitt 31 des Betätigungselements 3 verbunden, der aus elektrisch leitendem Material, vorzugsweise Metall, gefertigt ist und distalseitig einen Aufnahmespalt 31' aufweist. Dieser ist so geformt, dass bei Aufnahme des proximalen Endes 34' in dem Aufnahmespalt 31' der Stangenabschnitt 31 koaxial an dem zylindrischen Ansatz 3' des Endabschnitts 30 bündig anschlägt. Dann werden die elektrisch leitenden Teile miteinander verschweißt, um sie gegeneinander zu fixieren. D. h., der Kopplungseinsatz 35 wird mit dem Flachprofilstück 33 an den Fügekanten zwischen der Hakenaussparung 35' und dem distalen Ende 34 miteinander verschweißt, und der Stangenabschnitt 31 mit dem Flachprofilstück 33 an den Fügekanten zwischen dem Aufnahmespalt 31' und dem proximalen Ende des Flachprofilstücks 33.

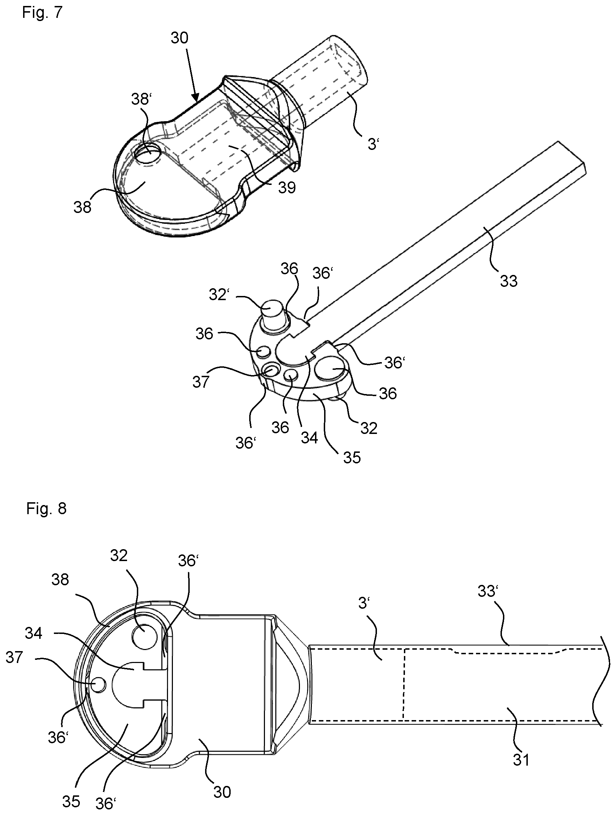

Wie in Fig. 7 zu sehen ist, ist der Kopplungseinsatz 35 an seiner Unterseite, die im gefügten Zustand auf dem Boden der Formaussparung 38 aufsitzt, mit mehreren Boden-Abstandshaltern 36 ausgebildet, so dass ein Spalt bzw. Zwischenraum mit definiertem Abstand zwischen der Unterseite des Kopplungseinsatzes 35 und dem Boden der Formaussparung 38 im Endabschnitt 30 gebildet wird. In der Darstellung ist ferner zu sehen, dass das distale Ende 34 des Flachprofilstücks 33 über die Unterseite des Kopplungseinsatzes 35 hinausragt und quasi ebenfalls als Boden-Abstandshalter in der Formaussparung 38 fungiert.

Außerdem weist der Kopplungseinsatz 35 an seiner umfänglichen Kontur drei Umfangs-Abstandshalter 36' auf, die beidseitig neben und gegenüber der Hakenaussparung 35' ausgebildet sind. So ist der Kopplungseinsatz 35 in der Formaussparung 38 zu deren Seitenwänden an drei Stellen, neben und gegenüber der Durchgangsöffnung 39, ausgerichtet und seine Position ist eindeutig festgelegt, wie insbesondere in Fig. 8 zu sehen ist. Auch hier verbleibt ein Spalt bzw. hier eine Fuge mit definierter Breite zwischen der umfänglichen Kontur des Kopplungseinsatzes 35 und den Seitenwänden der Formaussparung 38 im Endabschnitt 30. Die durch die Boden- und Umfangs-Abstandshalter 36, 36' verbleibenden Spalte stellen Klebehohlräume für die anschließende Verklebung bereit.

Zur Verklebung weist der Kopplungseinsatz 35 eine durchgehende Füllbohrung 37 auf, an der eine Klebespritze oder ein anderes Dosiersystem angesetzt werden kann, um Klebstoff durch die Füllbohrung 37 in den Zwischenraum zwischen der Unterseite des Kopplungseinsatzes 35 und dem Boden der Formaussparung 38 im Endabschnitt 30 einzudrücken. Bei kontinuierlicher Zufuhr von Klebstoff durch die Füllbohrung 37 verteilt sich der Klebstoff in dem Zwischenraum, der durch die Boden-Abstandshalter 36 definiert wird, bis zu den Seitenwände der Formaussparung 38 und steigt dort in den Fugen zwischen der umfänglichen Kontur des Kopplungseinsatzes 35 und den Seitenwänden der Formaussparung 38 auf, bis der komplette Kopplungseinsatz 35 erkennbar verklebt ist und alle Spalte mit Klebstoff gefüllt sind. Durch die Klebstofffüllung in den Spalten des Verbundbauteils werden dort Schmutzansammlungen im Gebrauch verhindert. Nach Aushärtung des Klebstoffs werden der zylindrische Ansatz 3' des elektrisch isolierenden Endabschnitts 30 und der Stangenabschnitt 31 des Betätigungselements 3, wie in Fig. 4 zu sehen, mit eine Isolatorhülle 33' überzogen, die die elektrische Isolation zum Außenschaft 7 herstellt.

Zur Übertragung der mechanischen Betätigungsbewegung des Betätigungselements 3 auf die Maulteile 2, 2' sind an dem scheibenförmigen Kopplungseinsatz 35 zwei Antriebszapfen 32, 32' ausgebildet. Der erste Antriebszapfen 32 befindet sich an der Oberseite des Kopplungseinsatzes 35 und ist dort in radialer Richtung zu der Längsachse L beabstandet. Der zweite Antriebszapfen 32', der an der Unterseite des Kopplungseinsatzes 35 ebenfalls radial beabstandet zur Längsachse L ausgebildet ist, ist, wie in Fig. 7 erkennbar, dazu vorgesehen, sich durch die Zapfenöffnung 38' zu erstrecken. Daher ist der Antriebszapfen 32' an der Unterseite des Kopplungseinsatzes 35 länger als der Antriebszapfen 32 an der Oberseite und weist ferner einen als Boden-Abstandshalter 36 dienenden Absatz auf. Bei dem fertigen Betätigungselement 3 sind die zwei Antriebszapfen 32, 32' rotationssymmetrisch bezüglich der Längsachse L an den voneinander abgewandten Seiten des laschenartigen Endabschnitts 30 angeordnet.

Jeder Antriebszapfen 32, 32' wird in einer Kopplungsöffnung 22 des jeweiligen Maulteils 2, 2' aufgenommen, um die Betätigungsbewegung des Betätigungselements 3 auf die Maulteile 2, 2' zu übertragen und das Drehmoment um die Schwenkachse S zu erzeugen. Durch den Verbundaufbau der Maulteile 2, 2'ist es dabei möglich, die beiden Stromübertragungswege zu den Wirkabschnitten 24, 24' der beiden Maulteile 2, 2' zu trennen. Dazu weist jedes Maulteil 2, 2', wie dargestellt in Fig. 9 bis 13, einen Betätigungsabschnitt 20, 20' auf, der aus einem elektrisch isolierenden Material, vorzugsweise aus Keramik, alternativ ggf. aus Kunststoff oder Glas, gefertigt ist. An jedem Betätigungsabschnitt 20, 20' ist ein Kopplungsabschnitt 25, 25' befestigt, der mit dem Wirkabschnitt 24, 24' verbunden ist und wie dieser aus einem elektrisch leitenden Material, beispielsweise Metall, gefertigt ist.

Der Kopplungsabschnitt 25 an dem Betätigungsabschnitt 20 des ersten Maulteils 2, abgebildet in Fig. 9 bis 11, verbindet den Wirkabschnitt 24 des ersten Maulteils 2 elektrisch leitend mit dem Betätigungselement 3, indem der Kopplungsabschnitt 25 des ersten Maulteils 2 den Kopplungseinsatz 35 des Betätigungselements 3 flächig kontaktiert. Der Betätigungsabschnitt 20 des ersten Maulteils 2 isoliert den Kopplungsabschnitt 25 und den Wirkabschnitt 24 des ersten Maulteils 2 von der Gabelhalterung 4. Also ist bei dem ersten Maulteil 2 der Kopplungsabschnitt 25 innenseitig und der Betätigungsabschnitt 20 außenseitig angeordnet. Beim zweiten Maulteil 2' ist es umgekehrt: Dessen Kopplungsabschnitt 25' ist außenseitig und der Betätigungsabschnitt 20 innenseitig angeordnet. Fig. 12 und 13 zeigen das zweite Maulteil 2', dessen Kopplungsabschnitt 25' den Wirkabschnitt 24' elektrisch leitend mit der Gabelhalterung 4 verbindet. Dabei kontaktiert der Kopplungsabschnitt 25' des zweiten Maulteils 2' die Innenfläche eines der Armabschnitte 50 flächig. Der Betätigungsabschnitt 20' des zweiten Maulteils 2' isoliert den Kopplungsabschnitt 25' und den Wirkabschnitt 24' von dem Betätigungselement 3.

Die Betätigungsabschnitte 20, 20' beider Maulteile 2, 2' sind laschenartig, d. h. flächig mit parallelen Seitenflächen und einem abgerundeten Ende, geformt und weisen - vom abgerundeten Ende abgewandt - einen Stutzen 29 auf, an dem der Wirkabschnitt 24, 24' zumindest teilweise bündig anschließt. Dazu ist der Stutzen 29 zumindest an der Außenseite des Betätigungsabschnitts 20, 20' zur Fortsetzung der Außenkontur des Wirkabschnitts 24, 24' geformt. Zur formschlüssigen Aufnahme der jeweiligen Kopplungsabschnitte 25, 25' weisen die laschenartigen Betätigungsabschnitte 20, 20' an einer Seitenfläche jeweils eine teilkreisförmige Formaussparung 28 auf. Eine damit verbundene Aussparung 29' in dem Stutzen 29 ist zur formschlüssigen Aufnahme eines Verbindungsabschnitts (unbezeichnet) zwischen dem Kopplungsabschnitt 25, 25' und dem Wirkabschnitt 24, 24' ausgebildet, wie in Fig. 10, 11 und 13 zu sehen ist.

Fig. 11 zeigt den transparent dargestellten Betätigungsabschnitt 20 (Isolatorbauteil des ersten Maulteils 2) getrennt von dessen Leiterbauteil aus Wirk- und Kopplungsabschnitt 24, 25. So ist zu erkennen, dass der Kopplungsabschnitt 25 an seiner Unterseite, die im gefügten Zustand auf dem Boden der Formaussparung 28 aufsitzt, mit mehreren Boden-Abstandshaltern 26 ausgebildet ist. So entsteht ein Spalt bzw. Zwischenraum mit definiertem Abstand zwischen der Unterseite des Kopplungsabschnitts 25 und dem Boden der Formaussparung 28 im Betätigungsabschnitt 20. Außerdem weist der Kopplungsabschnitt 25 an seiner Kontur drei Umfangs-Abstandshalter 26' auf, die benachbart zu und gegenüber dem Verbindungsabschnitt (vgl. Fig. 10) ausgebildet sind. So ist der Kopplungsabschnitt 25 in der Formaussparung 28 zu deren Seitenwänden an drei Stellen, neben und gegenüber der Verbindungsaussparung 29', ausgerichtet und seine Position ist eindeutig festgelegt. Auch hier verbleibt ein Spalt bzw. hier eine Fuge mit definierter Breite zwischen der umfänglichen Kontur des Kopplungsabschnitts 25 und den Seitenwänden der Formaussparung 28 im Betätigungsabschnitt 20.

Die durch die Boden- und Umfangs-Abstandshalter 26, 26' gebildeten Spalte definieren Klebehohlräume für die anschließende Verklebung. Zur Verklebung weist der Kopplungsabschnitt 25 eine durchgehende Füllbohrung 27 auf, an der eine Klebespritze oder ein anderes Dosiersystem angesetzt werden kann, um Klebstoff durch die Füllbohrung 27 in den Zwischenraum zwischen der Unterseite des Kopplungsabschnitts 25 und dem Boden der Formaussparung 28 im Betätigungsabschnitt 30 einzudrücken. Bei kontinuierlicher Zufuhr von Klebstoff durch die Füllbohrung 27 verteilt sich der Klebstoff in dem Zwischenraum, der durch die Boden-Abstandshalter 26 definiert wird, bis zu den Seitenwände der Formaussparung 28 und steigt dort in den Fugen zwischen der umfänglichen Kontur des Kopplungsabschnitts 25 und den Seitenwänden der Formaussparung 28 auf, bis der komplette Kopplungsabschnitt 25 erkennbar verklebt ist und alle Spalte mit Klebstoff gefüllt sind. Durch die Klebstofffüllung in den Spalten des Verbundbauteils werden dort Schmutzansammlungen im Gebrauch verhindert.

Entsprechendes gilt auch für das zweite Maulteil 2': Auch dort weist der Kopplungsabschnitt 25' entsprechende Boden-Abstandshalter an seiner Unterseite und, wie in Fig. 13 zu sehen, Umfangs-Abstandshalter 26' an der Kontur auf, sodass die zu der Formaussparung 28 entstehenden Spalte Klebehohlräume für einen durch die Füllbohrung 27 zugeführten Klebstoff bereitstellen.

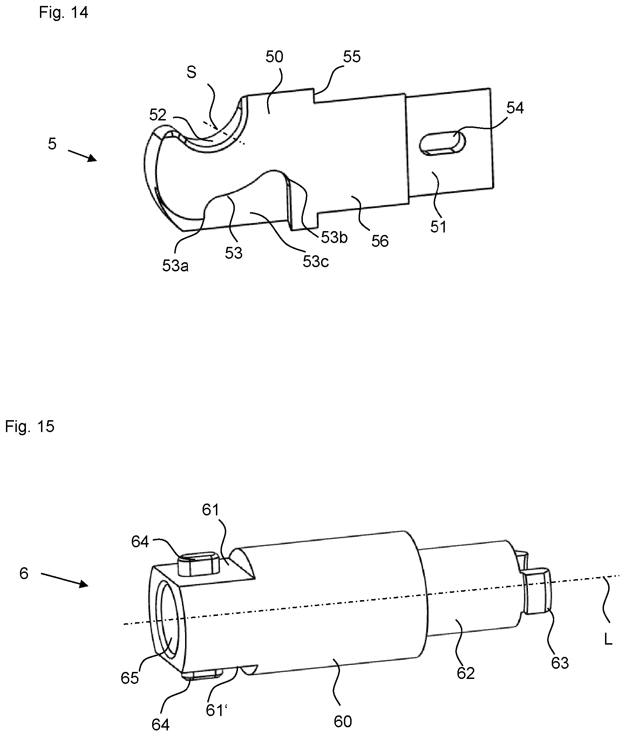

Die Montage der schwenkbar in der Gabelhalterung 4 gelagerten Maulteile 2, 2' wird durch Verwendung einer mehrteiligen Gabelhalterung 4 ermöglicht. Diese besteht aus zwei identisch ausgeführten Gabelarmen 5 und einer Gabelhalterung 6, für die in Fig. 14 und 15 ein Beispiel gezeigt ist. Die Verwendung der mehrteiligen Gabelhalterung 4 ermöglicht eine Sicherung der Maulteile 2, 2' gegen Herausfallen nach dem Fügen in der Endmontage, wobei ein Offenanschlag zur Begrenzung des Öffnungswinkels der Maulteile 2, 2'und eine hohe Kraftübertragung ohne den durchgehenden Führungsbolzen aus dem Stand der Technik realisiert werden. Jeder Gabelarm 5 stellt für jeweils eines der Maulteile 2, 2' nicht nur dessen schwenkbare Lagerung, sondern auch eine Zapfen-Bahnsteuerung bereit.

Für die schwenkbare Lagerung der Maulteile 2, 2' um die Schwenkachse S weist jeder Gabelarm 5 an dem Armabschnitt 50 eine Schwenkausnehmung 52 auf, in der der Schwenkzapfen 21 des jeweiligen Maulteils 2, 2' schwenkbar gelagert wird. Die Schwenkausnehmung 52 ist im Randbereich des Armabschnitts 50 angeordnet und dabei einseitig zum Rand hin geöffnet ausgebildet. Für die Zapfen-Bahnsteuerung ist auf der Innenseite des Armabschnitts 50 des Gabelarms 5 in Fig. 14 eine zu der durch die Schwenkausnehmung 52 definierte Schwenkachse S konzentrische Führungsbahn 53 als Steuerungskurve ausgebildet. Darin wird ein Führungszapfen 23 des jeweiligen Maulteils 2, 2' bei dessen Schwenkbewegung geführt. Der Führungszapfen 23 erstreckt sich parallel zum Schwenkzapfen 21 und weist in die gleiche Richtung. Die Führungsbahn 53 wird durch einen zylindrischen Flächenabschnitt 53 bereitgestellt, der Teil der Umrandung einer Aussparung 53c auf der Innenseite des Armabschnitts 50 ist, wobei die Schwenkachse S der Mittelachse der Zylinderform der Führungsbahn 53 entspricht.

Der Abstand der Führungsbahn 53 von der Schwenkausnehmung 52 entspricht dem Abstand des Führungszapfens 23 von dem Schwenkzapfen 21. Die Führungsbahn 53 weist beidseitig einen Anschlag 53a, 53b auf, an dem die Umrandung der Aussparung 53c von dem zylindrischen Verlauf abweicht und im gezeigten Beispiel radialparallel zur Längsachse L verläuft. Dabei ist Aussparung 53c zum Rand des Armabschnitts 50 in entgegengesetzter Richtung zur Schwenkausnehmung 52 geöffnet. In einer nicht dargestellten alternativen Ausführung kann die Aussparung 53c eine kreisbogenförmige Nut sein. In jedem Fall begrenzt der distale Anschlag 53a als Offenanschlag den Öffnungswinkel der Maulteile 2, 2'. Somit wird der maximale Öffnungswinkel der Maulteile 2, 2' durch die Winkelposition der Führungsbahn 53 festgelegt. Der proximale Anschlag 53b entspricht der Schließstellung der Maulteile 2, 2' bzw. kann z. B. als Kraftbegrenzungselement bei geschlossenen Maulteilen 2, 2' dienen.

Für die beiden getrennten Stromübertragungswege unterscheidet sich daher der Verbundaufbau der beiden Maulteile 2, 2' an dem elektrisch leitenden Kopplungsabschnitt 25, 25' und dem elektrisch isolierenden Betätigungsabschnitt 20, 20' im Hinblick auf die Anordnung der Kopplungsöffnung 22 einerseits und des Schwenk- und Führungszapfens 21, 23 andererseits.

So ist bei dem ersten Maulteil 2, dessen Wirkabschnitt 24 elektrisch mit dem Betätigungselement 3 verbunden wird, die Kopplungsöffnung 22 in dem Kopplungsabschnitt 25 (Fig. 10) ausgebildet. Darin wird der erste Antriebszapfen 32 am Endabschnitt 30 des Betätigungselements 3 aufgenommen, sodass der Kopplungsabschnitt 25 flächig die Oberseite des Kopplungseinsatzes 35 kontaktiert. Durch die flächige Kontaktierung ist der elektrische Übergangswiderstand bei der elektrisch leitenden Verbindung des Wirkabschnitts 24 des ersten Maulteils 2 mit dem Betätigungselement 3 gering.

In der in Fig. 9 gezeigten Ansicht des ersten Maulteils 2 ist die von dem Kopplungsabschnitt 25 abgewandten Seitenfläche des laschenartigen Betätigungsabschnitts 20 zu sehen, an der der Schwenkzapfen 21 und der Führungszapfen 23 ausgebildet sind. Der Betätigungsabschnitt 20 mit dem Schwenkzapfen 21 und dem Führungszapfen 23 aus dem elektrisch isolierenden Material sorgt damit an der gesamten Gelenkoberfläche für die elektrische Isolierung zur Gabelhalterung 4.

Bei dem zweiten Maulteil 2', dessen Wirkabschnitt 24' mit der Gabelhalterung 4 elektrisch verbunden wird, sind der Schwenkzapfen 21 und der Führungszapfen 23 an dem Kopplungsabschnitt 25' ausgebildet, der in der Formaussparung 28' des Betätigungsabschnitts 20' aufgenommen ist, wie in Fig. 13 zu sehen ist. Bei Aufnahme des Schwenkzapfens 21 und des Führungszapfens 23 in der Schwenkausnehmung 52 und der Führungsbahn 53 des Gabelarms 5 kommt der Kopplungsabschnitt 25' flächig mit der Innenseite des Gabelarms 5 in Kontakt. So wird auch hier der elektrische Übergangswiderstand bei der elektrisch leitenden Verbindung des Wirkabschnitts 24' des zweiten Maulteils 2' mit der Gabelhalterung 4 gering gehalten.

Fig. 12 zeigt das zweite Maulteil 2' mit der von dem Kopplungsabschnitt 25' abgewandten Seitenfläche des laschenartigen Betätigungsabschnitts 20', an der die Kopplungsöffnung 22 für den zweiten Antriebszapfen 32' des Betätigungselements 3 ausgebildet ist. Seitenwand und Boden der Kopplungsöffnung 22 bestehen somit aus dem elektrisch isolierenden Material des Betätigungsabschnitts 20'. Um eine ausreichende Wandstärke des Betätigungsabschnitts 20' zwischen der Kopplungsöffnung 22 auf der einen und der Formaussparung 28' auf der anderen Seitenfläche sicherzustellen, weicht die Formaussparung 28' von der Kreisform ab und erstreckt sich nicht über den Bereich der Kopplungsöffnung 22, der in Fig. 13 gestrichelt markiert ist. So ist das zweite Maulteil 2' an der gesamten Eingriffsoberfläche zu dem Endabschnitt 30 des Betätigungselements 3 elektrisch isoliert.

Für einen möglichst großen Hebel zum Schwenken jedes Maulteils 2, 2' sind die Kopplungsöffnung 22 auf der einen Seite und der Schwenkzapfen 21 auf der anderen Seite an dem jeweiligen Betätigungsabschnitt 20, 20' bzw. Kopplungsabschnitt 25, 25' möglichst weit voneinander beabstandet. Wie in Fig. 13 anhand der Strichelierung zu erkennen ist, die den Bereich für die Kopplungsöffnung 22 markiert, sind im vorliegenden Beispiel die Kopplungsöffnung 22 und der Schwenkzapfen 21 an gegenüberliegenden Randbereichen des Betätigungsabschnitts 20' angeordnet. Dasselbe gilt für den Betätigungsabschnitt 20 des ersten Maulteils 2, wie aus Fig. 9 und 10 entnehmbar ist. Entsprechend sind die Antriebszapfen 32, 32' des Kopplungseinsatzes 35 am distalen Endabschnitt 30 des Betätigungselements 3 von der Längsachse L beabstandet. So erstreckten sich die Antriebszapfen 32, 32' orthogonal zu der Längsachse L und in entgegengesetzte Richtungen von dem Randbereich auf der jeweiligen Seite des laschenartigen Endabschnitts 30. Ferner ist die zum Rand hin geöffnete Schwenkausnehmung 52 des Gabelarms 5, wie in Fig. 14 zu sehen, passend zu dem randständig an dem Betätigungsabschnitt 20, 20' angeordneten Schwenkzapfen 21 von der Längsachse L bzw. einer dazu parallelen Achse radial beabstandet.

Figur 4 zeigt ein Montageset für das chirurgische Bipolar-Maulteilwerkzeug 1, das die zwei Maulteile 2, 2', ein Betätigungselement 3, zwei Gabelarme 5 und einen Gabelkörper 6 umfasst. Das Betätigungselement 3 ist dabei bereits in den Gabelkörper 6 eingeführt. Die Gabelarme 5 und der Gabelkörper 6 bilden in einer Fügeanordnung miteinander die Gabelhalterung 4, die bei der Montage des chirurgischen Bipolar-Maulteilwerkzeugs 1 entsteht.

Der dreiteilige Aufbau der Gabelhalterung 4 aus den zwei Gabelarmen 5 und dem Gabelgrundkörper 6 gestattet die Montage des chirurgischen Maulteilwerkzeugs 1, das die vorbeschriebene Gelenkmechanik mit der Zapfen-Bahnsteuerung aufweist. In der Fügeanordnung der Gabelarme 5 mit dem Gabelgrundkörper 6 ist trotz der einseitig offenen Schwenkausnehmung 52 ein Verschieben oder Herausfallen der Maulteile 2, 2' in seitlicher Richtung orthogonal zur Schwenkachs S und zur Längsachse L nicht mehr möglich. Denn der Führungszapfen 23 des Maulteils 2, 2' steht immer im Eingriff in der Führungsbahn 53 des Armabschnitts 50 der Gabelhalterung 4.

Wie in Fig. 14 und 15 zu sehen ist, weist jeder Gabelarm 5 außer dem Armabschnitt 50 einen Fügeabschnitt 51 auf, und der Gabelkörper 6 hat zwei Anlageabschnitte 61, 61', die jeweils zur Verbindung mit einem Fügeabschnitt 51 eines Gabelarms 5 ausgebildet sind, um bei der Montage die Herstellung der Fügeanordnung zu erleichtern. Die Anlageabschnitte 61, 61' liegen diametral an dem Rohrabschnitt 60 des Gabelkörpers 6 und sind im Wesentlichen flächig ausgebildet. Dabei erstrecken sie sich die Anlageabschnitte 61, 61' als zylindersegmentförmige Ausnehmung zu dem distalen Ende des Gabelkörpers 6. Der Fügeabschnitt 51 ist entsprechend als Zylindersegment geformt, sodass in der Fügeanordnung der Fügeabschnitt 51 der Gabelarme 5 die Anlageabschnitte 61, 61' des Gabelgrundkörpers 6 bündig zur Zylinderform des Rohrabschnitts 60 ergänzt.

Mit der komplementären Formgebung von Fügeabschnitt 51 und Anlageabschnitt 61, 61' wird die korrekte Ausrichtung der Armabschnitte 50 parallel zur Längsachse L bei der Anordnung der Gabelarme 5 an dem Gabelkörper 6 erleichtert. Unterstützt wird dies durch Formelemente 54 an jedem Fügeabschnitt 51, die mit komplementär gestalteten Gegenformelementen 64 am Anlageabschnitt 61, 61' formschlüssig in Eingriff gebracht werden können. In dem gezeigten Beispiel ist das Formelement 54 am Fügeabschnitt 51 des Gabelarms 5 ein Langloch 54. Das entsprechende Gegenformelement 64 an den Anlageabschnitten 61, 61' des Gabelkörpers 6 ist als Passfederprofil 64 ausgebildet, das aus der Fläche des Anlageabschnitts 61, 61' herausragt. Das Langloch 54 bildet mit dem Passfederprofil 64 ein Formschluss-Paar, wobei das im vorliegenden Beispiel parallel zur Längsachse L ausgerichtet ist.

Die Anlagenabschnitte 61, 61' des Gabelkörpers 6 mit den Passfederprofilen 64 sind rotationssymmetrisch ausgebildet und die Gabelarme 5 mit den Langlöchern 54 an den Fügeabschnitten 51 sind gleichartig gefertigt, sodass keine Unterscheidung der Gabelarme 5 bei der Anordnung an dem Gabelkörper 6 erforderlich ist.

Durch die mehrteilige Gabelhalterung 4 kann nicht nur auf den durchgehenden Führungsbolzen verzichtet werden, sondern auch auf die dafür erforderliche Durchgangsöffnung in den Armabschnitten 50 der Gabelhalterung 4, die die Außenoberfläche der Gabelhalterung unterbricht. Die daher geschlossene Außenoberfläche der mehrteiligen Gabelhalterung 4 stellt sicher, dass keine Gelenkteile über die Außenoberfläche hinausragen können. Außerdem wird durch den Verzicht auf den durchgehenden Führungsbolzen aus dem Stand der Technik bei dem chirurgischen Bipolar-Maulteilwerkzeug 1 eine Kriechstreckenverkürzung vermieden.

Nach der Fertigung der Verbundbau-Maulteile 2, 2' und des Verbundbau-Betätigungselements 3 zur Bereitstellung des Montagesets entsprechend Fig. 4 in Schritt S0 des Herstellungsverfahrens erfolgt im nächsten Schritt S1 die Montage zu einem chirurgischen Bipolar-Maulteilwerkzeug 1. In Fig. 4 ist bereits das Betätigungselement 3 in den Gabelkörper 6 eingeführt dargestellt. Dabei wird ein länglicher Stangenabschnitt 31 des Betätigungselements 3, der sich von dem distalen Endabschnitt 30 erstreckt, durch eine zur Längsachse L koaxiale Durchgangsbohrung 65 des Gabelkörpers 6 geführt (Fig. 15). Der distale Endabschnitt 30 des Betätigungselements 3 befindet sich dann auf distaler Seite des Gabelköpers 6, an der sich die Anlageabschnitte 61, 61' befinden. Der Stangenabschnitt 31 ragt proximalseitig aus dem Gabelköper 6 hinaus.

Im nächsten Verfahrensschritt S2 wird das erste Maulteil 2 an einem ersten Gabelarm 5 gelagert, wobei die an dem Betätigungsabschnitt 20 ausgebildeten Schwenk- und Führungszapfen 21, 23 jeweils in der u-förmigen, einseitig offenen Schwenkausnehmung 52 und in der Aussparung 53c an der Führungsbahn 53 des Gabelarms 5 aufgenommen werden, sodass das erste Maulteil 2 um seine Schwenkachse S schwenken kann.

Im folgenden Verfahrensschritt S3 wird der Kopplungseinsatz 35 am distalen Endabschnitt 30 des in den Gabelkörper 6 eingeführten Betätigungselements 3 mit dem ersten Maulteil 2 in Eingriff gebracht. Dabei wird der erste Antriebszapfen 32, der an der Oberseite des Kopplungseinsatzes 35 ausgebildet ist, in der Kopplungsöffnung 22 an dem Kopplungsabschnitt 25 des ersten Maulteils 2 aufgenommen. Damit kann eine Axialbewegung des Betätigungselements 3 in eine Schwenkbewegung des Maulteils 2 übertragen werden, wobei der flächige Kontakt des Kopplungseinsatzes 35 an dem Kopplungsabschnitt 25 eine elektrische Verbindung des Betätigungselements 3 und des Wirkabschnitts 24 mit geringem Übergangswiderstand bereitstellt. Ferner erfolgt in diesem Schritt S3 der Eingriff des Gabelkörpers 6 mit dem ersten Gabelarm 5. Dazu wird der erste Gabelarm 5 mit dem Fügeabschnitt 51 an dem ersten Anlageabschnitt 61 des Gabelkörpers 6 angeordnet, wobei ein formschlüssiger Eingriff des Formelements 54, hier Langloch, mit dem Gegenformelement 64 des Anlageabschnitts 61, hier Passfederprofil, stattfindet. Der formschlüssige Eingriff sorgt für die korrekte Ausrichtung des ersten Gabelarms 5 an dem Gabelkörper 6.

Anschließend wird mit Verfahrensschritt S4 das zweite Maulteil 2' mit dem Betätigungselement 3 in Eingriff gebracht. Dabei wird der zweite Antriebszapfen 32', der durch die Zapfenöffnung 38' aus dem elektrisch isolierenden Endabschnitt 30 des Betätigungselements 3 ragt, in der Kopplungsöffnung 22 am Betätigungsabschnitt 20' des zweiten Maulteils 2' aufgenommen. Durch das elektrisch isolierende Material des Betätigungsabschnitts 20' des zweiten Maulteils 2' ist der zweite Antriebszapfen 32' des Betätigungselements 3 elektrisch von dem Wirk- und Kopplungsabschnitt 24', 25' des zweiten Maulteils 2' getrennt.

Verfahrensschritt S5 betrifft die Anordnung des zweiten Gabelarms 5, wobei der an dem Kopplungsabschnitt 25' des zweiten Maulteils 2' ausgebildete Schwenkzapfen 21 in der Schwenkausnehmung 52 des zweiten Gabelarms 5 aufgenommen und gelagert wird. Gleichzeitig wird dabei der ebenfalls an dem Kopplungsabschnitt 25' ausgebildete Führungszapfen 23 in der Führungsbahn 53 des zweiten Gabelarms 5 aufgenommen. Dabei kommt der Kopplungsabschnitt 25' des zweiten Maulteils 2' flächig in Kontakt mit der Innenseite des Gabelarms 5 für eine widerstandsarme elektrische Verbindung. Die ausgerichtete Anordnung des zweiten Gabelarms 5 an dem Gabelkörper 6 umfasst den formschlüssigen Eingriff des Langlochs 54 im Fügeabschnitt 51 des zweiten Gabelarms 5 mit dem Passfederprofil 64 am zweiten Anlageabschnitt 61' des Gabelkörpers 6.

Zur Bildung der Gabelhalterung 4 in der Fügeanordnung der beiden Gabelarme 5 und des Gabelkörpers 6 werden die Fügeabschnitte 51 mit den Anlageabschnitten 61, 61' stoffschlüssig verbunden, sodass mit Verfahrensschritt S6 das chirurgische Bipolar-Maulteilwerkzeug 1 aus Fig. 1 erhalten wird. Zum stoffschlüssigen Verbinden kann beispielsweise jedes Formschluss-Paar aus Langloch 54 und Passfederelement 64 an den Verbindungskanten der Wirkflächen verschweißt werden, die aufgrund der durchgehenden Langlöcher 54 von außen zugänglich sind. Alternativ oder zusätzlich können auch die weiteren Verbindungskanten zwischen den Gabelarmen 5 und dem Gabelkörper 5 stoffschlüssig verbunden, z. B. verschweißt werden. Die weiteren Verbindungskanten befinden sich an der zylindrischen Außenoberfläche des Gabelkörpers 6, die die Anlageabschnitte 61, 61' als zylindersegmentförmigen Ausnehmungen begrenzen, und an der zylindrischen Außenoberfläche der zylindersegmentförmigen Fügeabschnitte 51. Anschließend kann die Außenoberfläche der Gabelhalterung 4 für ein homogenes Erscheinungsbild nochmals überschliffen werden.

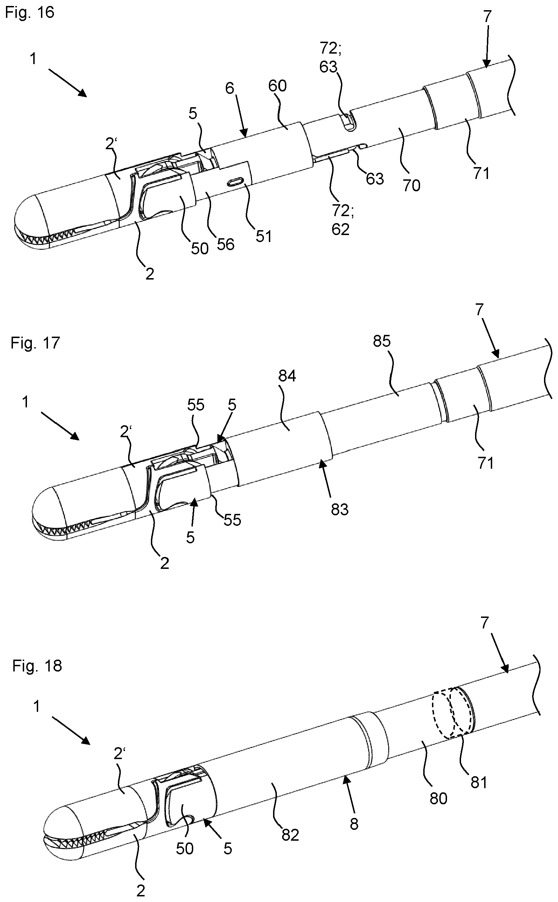

Zur weiterführenden Montage des chirurgischen Maulteilwerkzeugs 1 zu dem chirurgischen Bipolar-Instrument 10 (Fig. 3) wird der mit der Isolatorhülle 33' elektrisch isolierte Stangenabschnitt 31 des Betätigungselements 3 in den Außenschaft 7 eingeführt. Der Außenschaft 7, der aus einem elektrisch leitenden Material besteht, wird an seinem distalen Ende mit der Gabelhalterung 4 verbunden. Dazu weist der Außenschaft 7 einen distalen Kopplungsabschnitt 70 auf, wie in Fig. 2 und 16 zu sehen ist. Der distale Kupplungsabschnitt 70 weist zwei Bajonettnuten 72 auf, die sich L-förmig vom offenen distalen Ende des Außenschafts 7 erstrecken. Der Gabelkörper 6 hat proximalseitig an dem Rohrabschnitt 60 einen abgesetzten, im Durchmesser reduzierten Kupplungsabschnitt 62 und zwei Bajonettnocken 63, wie in Fig. 15, zu sehen ist. Bei Aufnahme des Kupplungsabschnitts 62 in den distalen Kopplungsabschnitt 70 des Außenschafts 7 treten die beiden Bajonettnocken 63 mit den zwei Bajonettnuten 72 in Eingriff, sodass das distale Ende des Außenschafts 7 an dem Ringabsatz anschlägt, der zwischen dem Rohrabschnitt 60 und dem Kupplungsabschnitt 62 ausgebildet ist. Andere Verbindungsarten zwischen einem Außenschaft und einer Gabelhalterung eines chirurgischen Bipolar-Instruments sind bekannt, und können alternativ zu der Bajonettverbindung eingesetzt werden.

Zur Sicherung der Verbindung der Gabelhalterung 4 mit dem Außenschaft 7 ist ferner eine Hülsenanordnung aus einer Haltehülse 83 und einer Verbindunghülse 8 (Fig. 17, 18) vorgesehen. Diese können aus einem elektrisch isolierenden Material bestehen. Die in Fig. 17 abgebildete Haltehülse 83 hat einen distalen Halteabschnitt 84, der den Rohrabschnitt 60 mit den an den Anlageabschnitten 61, 61' angeordneten Fügeabschnitten 51 der Gabelarme 5 umgibt. Ein von dem distalen Halteabschnitt 84 abgesetzter, im Durchmesser reduzierter proximaler Halteabschnitt 85 der Haltehülse 83 umgibt den distalen Kupplungsabschnitt 70 des Außenschafts 7, in dem der Kupplungsabschnitt 62 des Gabelkörpers 6 aufgenommen ist. Dazu sind die Innendurchmesser des distalen und proximalen Halteabschnitts 84, 85 jeweils entsprechend an die Außendurchmesser des Rohrabschnitts 60 des Gabelkörpers 6 und des distalen Kupplungsabschnitts 70 des Außenschafts 7 angepasst.

Die Verbindungshülse 8 in Fig. 18 umgibt die Haltehülse 83 und erstreckt sich beidseitig darüber hinaus, wobei die Verbindungshülse 8 mit einem distalen Anlageabschnitt 82 an einem radialen Absatz 55 an der teilzylindrisch geformten Außenseite der Gabelarme 5 bündig anschlägt. So ist die Kontur der distalen Werkzeugspitze stetig, glatt und ohne radiale Vorsprünge. Dabei unterteilt der an der Außenseite gebildete radiale Absatz 55 die Gabelarme 5 in den Armabschnitt 50 und einen Zwischenabschnitt 56, an den sich der Fügeabschnitt 51 anschließt (vgl. Fig. 14, 17). Hierbei entspricht der Außendurchmesser des distalen Anlageabschnitts 82 der Verbindungshülse 8 einem Außendurchmesser der teilzylindrischen Außenseite der Armabschnitte 50, während der davon abgesetzte Außendurchmesser der teilzylindrischen Außenseite des Zwischenabschnitts 56 dem Außendurchmesser der teilzylindrischen Außenseite des Fügeabschnitts 51 entspricht.

Anderenends weist die Verbindungshülse 8 einen proximalen Verbindungsabschnitt 80 mit einem Innengewinde 81 als Verbindungselement 81 zum Eingriff mit einem Außengewinde 71 auf, das der Außenschaft 7 als zusätzliches Verbindungselement 71 aufweist. Das Außengewinde 71 befindet sich proximalseitig des distalen Kupplungsabschnitts 70, sodass es nicht von der Haltehülse 83 bedeckt wird, (vgl. Fig. 16, 17). Dabei ist eine Innenkontur der Verbindungshülse 8 an die Außenkontur der Haltehülse 83 angepasst. Der proximale Verbindungsabschnitt 80 ist dabei radial von dem distalen Anschlagsabschnitt 82 der Verbindungshülse 8 abgesetzt, d. h. mit reduzierten Außendurchmesser ausgebildet. Zur weiteren Abdichtung und zur Glättung der Außenkontur der distalen Werkzeugspitze wird eine Dichthülle 7', z. B. ein Schrumpfschlauch, angeordnet (Fig. 3 dargestellt), die / der sich entlang dem Außenschaft 7 und dem proximale Verbindungsabschnitt 80 erstreckt, um bündig mit der Verbindungshülse 8 abzuschließen.

Je nach Durchmesserverhältnis zwischen den Hülsen 8, 83 und dem distalen Kupplungsabschnitt 70, dem Verbindungselement 71 und dem Außenschaft 7 kann zur Montage vorgesehen sein, dass die Haltehülse 83 mit ihren distalen Halteabschnitt 84 auf dem Rohrabschnitt 60 und ggf. auch die Verbindungshülse 8 ihrem distalen Anlageabschnitt 82 auf der Haltehülse 8 angeordnet werden, bevor der Kupplungsabschnitt 62 des Gabelkörpers 6 mit dem distalen Kupplungsabschnitt 70 des Außenschafts 7 verbunden wird. Zur Kopplung des Außenschafts 7 mit der Gabelhalterung 4 wird dann der distale Kupplungsabschnitt 70 in den proximalen Halteabschnitt 85 der Haltehülse 83 innerhalb des proximalen Verbindungsabschnitts 80 der Verbindungshülse 8 eingeführt, wobei die Bajonettnocken 63 am Kupplungsabschnitt 62 des Gabelkörpers 6 in die Bajonettnuten 72 des distalen Kupplungsabschnitts 70 aufgenommen und das Innengewinde 81 am Verbindungsabschnitt 80 mit dem Außengewinde 71 am Außenschaft 7 in Eingriff gebracht werden.

Zur Fertigstellung des chirurgischen Bipolar-Instruments 10 wird der Außenschaft 7 wird an seinem proximalen Ende mit der Betätigungseinrichtung 9 verbunden, die mit dem proximalen Ende des Betätigungselements 3 in Eingriff gebracht wird. Die Betätigungseinrichtung 9 weist ein bipolares Anschlusselement 91 auf, über das der Außenschaft 7 und das Betätigungselement 3 mit einem Bipolar-Generator (nicht abgebildet) verbindbar sind.

Die vorliegende Erfindung stellt chirurgisches Bipolar-Maulteilwerkzeug 1, ein Verfahren zu seiner Herstellung und ein entsprechendes Bipolar-Instrument 10 bereit. Das Bipolar-Maulteilwerkzeug 1 ist mit einem ersten Verbundbau-Maulteil 2, das einen ersten Wirkabschnitt 24 aus einem Leitermaterial und einen ersten Betätigungsabschnitt 20 aus einem Isolatormaterial aufweist, und mit einem zweiten Verbundbau-Maulteil 2', das einen zweiten Wirkabschnitt 24' aus einem Leitermaterial und einen zweiten Betätigungsabschnitt 20' aus einem Isolatormaterial aufweist ausgestattet, und mit einem Verbundbau-Betätigungselement 3 aus einem Leitermaterial und einem Isolatormaterial, sowie einer Gabelhalterung 4 aus einem Leitermaterial, die zwei Armabschnitte 50 und zumindest einen Rohrabschnitt 60 aufweist, durch den sich das Verbundbau-Betätigungselement 3 zum Eingriff mit den beiden Verbundbau-Maulteilen 2, 2' erstreckt, die um jeweils eine Schwenkachse S beweglich zwischen den Armabschnitten 50 gelagert sind. Dabei ist der Wirkabschnitt 24 des ersten Verbundbau-Maulteils 2 mit dem Verbundbau-Betätigungselement 3 elektrisch verbunden und durch den ersten Betätigungsabschnitt 20 von der Gabelhalterung 4 elektrisch isoliert, und der zweite Wirkabschnitt 24' des zweiten Verbundbau-Maulteils 2' ist mit der Gabelhalterung 4 elektrisch verbunden und durch den zweiten Betätigungsabschnitt 20 von dem Verbundbau-Betätigungselement 3 elektrisch isoliert ist. Das Verbundbau-Betätigungselement 3 und die Gabelhalterung 4 sind elektrisch voneinander isoliert. Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

### BEZUGSZEICHENLISTE

- 1: Chirurgisches Bipolar-Maulteilwerkzeug
- 2, 2': Verbundbau-Maulteil
- 20, 20': Betätigungsabschnitt
- 21: Schwenkzapfen
- 22: Kopplungsöffnung
- 23: Führungszapfen
- 24, 24': Wirkabschnitt
- 25, 25': Kopplungsabschnitt
- 26, 26': Boden-, Umfangs-Abstandshalter
- 27: Füllbohrung
- 28, 28': Formaussparung
- 29, 29': Stutzen, Aussparung
- 3, 3': Verbundbau-Betätigungselement, zylindrischer Ansatz
- 30, 30': Distaler Endabschnitt, keilartiger Abschnitt
- 31, 31': Stangenabschnitt, Aufnahmespalt
- 32, 32': Antriebszapfen
- 33, 33': Flachprofilstück, Isolatorhülle
- 34, 34': Distales Ende/Hakenstruktur, proximales Ende
- 35, 35': Kopplungseinsatz, Aussparung
- 36, 36': Boden-, Umfangs-Abstandshalter
- 37: Füllbohrung
- 38, 38': Formaussparung, Zapfenöffnung
- 39: Durchgangsöffnung
- 4: Gabelhalterung
- 5: Gabelarm
- 50: Armabschnitt
- 51: Fügeabschnitt
- 52: Schwenkausnehmung
- 53: Führungsbahn
- 53a, b, c: Distaler, proximaler Anschlag, Aussparung
- 54: Formelement/Langloch
- 55: Radialer Absatz
- 56: Zwischenabschnitt
- 6: Gabelkörper
- 60: Rohrabschnitt
- 61, 61': Anlageabschnitt
- 62: Abgesetzter, proximaler Kupplungsabschnitt
- 63: Kupplungselement/Bajonettnocken
- 64: Gegenformelement/Passfederprofil
- 7: Außenschaft
- 70: Distaler Kupplungsabschnitt
- 71: Verbindungselement/Außengewinde
- 72: Kupplungselement/Bajonettnut
- 7': Dichthülle/Schrumpfschlauch
- 8: Verbindungshülse
- 80: Proximaler Verbindungsabschnitt
- 81: Verbindungselement/Innengewinde
- 82: Distaler Anlageabschnitt
- 83: Haltehülse
- 84: Distaler Halteabschnitt
- 85: Proximaler Halteabschnitt
- 9: Betätigungseinrichtung
- 91: Bipolares Anschlusselement
- 10: chirurgisches Bipolar-Instrument
- S: Schwenkachse
- L: Bipolar-Instrumentenlängsachse

## Patentansprüche

1. Chirurgisches Bipolar-Maulteilwerkzeug (1) mit
- einem ersten Verbundbau-Maulteil (2), das einen ersten Wirkabschnitt (24) aus einem Leitermaterial und einen ersten Betätigungsabschnitt (20) aus einem Isolatormaterial aufweist, und
- einem zweiten Verbundbau-Maulteil (2'), das einen zweiten Wirkabschnitt (24') aus einem Leitermaterial und einen zweiten Betätigungsabschnitt (20') aus einem Isolatormaterial aufweist, und
- einem Verbundbau-Betätigungselement (3) aus einem Leitermaterial und einem Isolatormaterial, und
- einer Gabelhalterung (4) aus einem Leitermaterial, die zwei Armabschnitte (50) und zumindest einen Rohrabschnitt (60) aufweist, durch den sich das Verbundbau-Betätigungselement (3) zum Eingriff mit den beiden Verbundbau-Maulteilen (2, 2') erstreckt, die um jeweils eine Schwenkachse (S) beweglich zwischen den Armabschnitten (50) gelagert sind, wobei
- der erste Wirkabschnitt (24) des ersten Verbundbau-Maulteils (2) mit dem Verbundbau-Betätigungselement (3) elektrisch verbunden und durch den ersten Betätigungsabschnitt (20) von der Gabelhalterung (4) elektrisch isoliert ist, und
- der zweite Wirkabschnitt (24') des zweiten Verbundbau-Maulteils (2') mit der Gabelhalterung (4) elektrisch verbunden und durch den zweiten Betätigungsabschnitt (20) von dem Verbundbau-Betätigungselement (3) elektrisch isoliert ist, und
- das Verbundbau-Betätigungselement (3) und die Gabelhalterung (4) elektrisch voneinander isoliert sind.

2. Chirurgisches Bipolar-Maulteilwerkzeug (1) nach Anspruch 1, wobei
das erste Verbundbau-Maulteil (2) einen ersten Kopplungsabschnitt (25) aus dem Leitermaterial aufweist, der mit dem ersten Wirkabschnitt (24) verbunden ist, wobei der erste Kopplungsabschnitt (25) und der erste Betätigungsabschnitt (20) ineinandergreifend verbunden sind, und
das zweite Verbundbau-Maulteil (2') einen zweiten Kopplungsabschnitt (25') aus dem Leitermaterial aufweist, der mit dem zweiten Wirkabschnitt (24') verbunden ist, wobei der zweite Kopplungsabschnitt (25') und der zweite Betätigungsabschnitt (20') ineinandergreifend verbunden sind, und
das Verbundbau-Betätigungselement (3) einen Kopplungseinsatz (35) aus dem Leitermaterial, der mit einem Stangenabschnitt (31) aus dem Leitermaterial verbunden ist, und einen distalen Endabschnitt (30) aus dem Isolatormaterial aufweist, wobei der Kopplungseinsatz (35) und der distale Endabschnitt (30) ineinandergreifend verbunden sind, wobei der Stangenabschnitt (31) mit einer Isolatorhülle (33') ummantelt ist, die sich an den distalen Endabschnitt (30) anschließt, wobei
die elektrische Verbindung des ersten Wirkabschnitts (24) mit dem Verbundbau-Betätigungselement (3) durch den ersten Kopplungsabschnitt (25) hergestellt wird, der den Kopplungseinsatz (35) kontaktiert, und die elektrische Isolierung des Verbundbau-Betätigungselements (3) von der Gabelhalterung durch den distalen Endabschnitt (30) und die Isolatorhülle (33') bereitgestellt wird.

3. Chirurgisches Bipolar-Maulteilwerkzeug (1) nach Anspruch 2, wobei
jeder Armabschnitt (50) eine Schwenkausnehmung (52) zur Bildung der jeweiligen Schwenkachse (S) aufweist, und
das erste Verbundbau-Maulteil (2)
- an dem ersten Betätigungsabschnitt (20) einen Schwenkzapfen (21) aufweist, der in der Schwenkausnehmung (52) eines ersten Armabschnitts (50) aufgenommen ist, und
- an dem ersten Kopplungsabschnitt (25) eine Kopplungsöffnung (22) aufweist, in der ein erster an dem Kopplungseinsatz (35) ausgebildeter Antriebszapfen (32) des Verbundbau-Betätigungselements (3) aufgenommen ist, und
das zweite Verbundbau-Maulteil (2')
- an dem zweiten Betätigungsabschnitt (20') eine Kopplungsöffnung (22) aufweist, in der ein zweiter an dem Kopplungseinsatz (35) ausgebildeter Antriebszapfen (32') des Verbundbau-Betätigungselements (3) aufgenommen ist, und
- an dem zweiten Kopplungsabschnitt (25') einen Schwenkzapfen (21) aufweist, der in der Schwenkausnehmung (52) eines zweiten Armabschnitts (50) aufgenommen ist.

4. Chirurgisches Bipolar-Maulteilwerkzeug (1) nach Anspruch 3, wobei
der Kopplungseinsatz (35) in einer Formaussparung (38) angeordnet ist, die in dem distalen Endabschnitt (30) ausgebildet ist und eine mit dem Kopplungseinsatz (35) abgestimmte Form aufweist, wobei
der erste Antriebszapfen (32) sich von einer freien, von dem distalen Endabschnitt (30) abgewandten Oberseite des Kopplungseinsatzes (35) erstreckt, und
der zweite Antriebszapfen (32') sich von einer dem distalen Endabschnitt (30) zugewandten Unterseite des Kopplungseinsatzes (35) durch eine in dem distalen Endabschnitt (30) ausgebildete Zapfenöffnung (38') erstreckt.

5. Chirurgisches Bipolar-Maulteilwerkzeug (1) nach zumindest einem der Ansprüche 2 bis 4, wobei die Verbindung des Kopplungseinsatzes (35) mit dem Stangenabschnitt (31) durch ein Flachprofilstück (33) aus dem Leitermaterial bereitgestellt wird, wobei ein distales Ende (34) des Flachprofilstücks (33) in einer Aussparung (35') ineinandergreifend aufgenommen ist, die in dem Kopplungseinsatz (35) ausgebildet ist, und ein proximales Ende (34') des Flachprofilstücks (33) in einem an dem distalen Ende des Stangenabschnitts (31) ausgebildeten Aufnahmespalt (31') aufgenommen ist.

6. Chirurgisches Bipolar-Maulteilwerkzeug (1) nach Anspruch 4 oder 5, wobei
der Kopplungseinsatz (35) an seiner Unterseite zumindest einen Boden-Abstandshalter (36) aufweist, durch den ein Zwischenraum zwischen der Unterseite des Kopplungseinsatzes (35) und einem Boden der Formaussparung (38) im Endabschnitt (30) gebildet wird,
wobei der Kopplungseinsatz (35) oder der distale Endabschnitt (30) eine Füllbohrung (37) aufweist, die eine Verbindung mit dem Zwischenraum bildet, der mit einem Klebstoff gefüllt wird.

7. Chirurgisches Bipolar-Maulteilwerkzeug (1) nach zumindest einem der Ansprüche 4 bis 6, wobei der Kopplungseinsatz (35) an seiner Kontur zumindest zwei Umfangs-Abstandshalter (36') aufweist, durch die der Kopplungseinsatz (35) in der Formaussparung (38) in Bezug zu deren Seitenwänden ausgerichtet und jeweils eine Fuge zwischen der Kontur des Kopplungseinsatzes (35) und den Seitenwänden der Formaussparung (38) ausgebildet wird, wobei die Fugen mit einem Klebstoff gefüllt sind.

8. Chirurgisches Bipolar-Maulteilwerkzeug (1) nach zumindest einem der Ansprüche 2 bis 6, wobei der erste Kopplungsabschnitt (25) in einer Formaussparung (28) angeordnet ist, die in dem ersten Betätigungsabschnitt (20) ausgebildet ist und eine mit dem ersten Kopplungsabschnitt (25) abgestimmte Form aufweist, und
der zweite Kopplungsabschnitt (25') in einer Formaussparung (28') angeordnet ist, die in dem zweiten Betätigungsabschnitt (20') ausgebildet ist und eine mit dem zweiten Kopplungsabschnitt (25') abgestimmte Form aufweist.

9. Chirurgisches Bipolar-Maulteilwerkzeug (1) nach Anspruch 8, wobei
jeder Kopplungsabschnitt (25, 25')
- an seiner Unterseite zumindest einen Boden-Abstandshalter (26) aufweist, durch den ein Zwischenraum zwischen der Unterseite des Kopplungsabschnitts (25, 25') und einem Boden der Formaussparung (28, 28') ausgebildet wird, wobei jeder Kopplungsabschnitt (25, 25') oder Betätigungsabschnitt (20, 20') eine Füllbohrung (27) aufweist, die mit dem Zwischenraum verbunden ist, der mit einem Klebstoff gefüllt wird,
und/oder
- an seiner Kontur zumindest zwei Umfangs-Abstandshalter (26') aufweist, durch die der Kopplungsabschnitt (25, 25') in der Formaussparung (28, 28') in Bezug zu deren Seitenwänden ausgerichtet und jeweils eine Fuge zwischen der umfänglichen Kontur des Kopplungsabschnitts (25, 25') und den Seitenwänden der Formaussparung (28, 28') ausgebildet wird, wobei die Fugen mit einem Klebstoff gefüllt sind.

10. Chirurgisches Bipolar-Maulteilwerkzeug (1) nach zumindest einem der Ansprüche 3 bis 9, wobei jedes Verbundbau-Maulteil (2, 2') einen Führungszapfen (23) und jeder Armabschnitt (50) eine zu der Schwenkausnehmung (52) konzentrische Führungsbahn (53) zur Führung des Führungszapfens (23) aufweist, dessen Abstand von dem Schwenkzapfen (21) einem Abstand der Führungsbahn (53) von der Schwenkausnehmung (52) entspricht.

11. Chirurgisches Bipolar-Maulteilwerkzeug (1) nach zumindest einem der Ansprüche 1 bis 10, wobei die Gabelhalterung (4) aus zwei Gabelarmen (5), die die Armabschnitte (50) aufweisen, und einem Gabelkörper (6), der den Rohrabschnitt (60) aufweist, in einer Fügeanordnung gebildet wird, wobei jeder Gabelarm (5) einen Fügeabschnitt (51) aufweist und der Gabelkörper (6) zwei Anlageabschnitte (61, 61') aufweist, die in der Fügeanordnung mit den Fügeabschnitten (51) der Gabelarme (5) durch eine Formschlussverbindung, eine Stoffschlussverbindung, eine Kraftschlussverbindung oder eine Kombination davon verbunden sind.

12. Chirurgisches Bipolar-Maulteilwerkzeug (1) nach Anspruch 11, wobei
der Gabelkörper (6) proximalseitig an dem Rohrabschnitt (60) einen abgesetzten, im Durchmesser reduzierten Kupplungsabschnitt (62) und/oder zumindest ein Kupplungselement (63) aufweist, das zur Verbindung mit einem Kuppelelement (72) an einem distalen Kupplungsabschnitt (70) eines Außenschafts (7) ausgebildet ist, in dem das Verbundbau-Betätigungselement (3) längsbeweglich anordenbar ist.

13. Chirurgisches Bipolar-Maulteilwerkzeug (1) nach Anspruch 12, wobei
eine Haltehülse (83) als Haltelement einen distalen Halteabschnitt (84), der den Rohrabschnitt (60) in der Fügeanordung umgibt, und einen proximalen Halteabschnitt (85) aufweist, der dazu ausgebildet ist, in einer Verbindungsanordnung des Gabelkörpers (6) und des Außenschafts (7) dessen distalen Kupplungsabschnitt (70) mit dem darin angeordneten Kupplungsabschnitt (62) aufzunehmen.

14. Chirurgisches Bipolar-Maulteilwerkzeug (1) nach Anspruch 12 oder 13, wobei das chirurgisches Maulteilwerkzeug (1) eine Verbindungshülse (8) aufweist, die die Verbindung der Gabelhalterung (4) mit dem Außenschaft (7) sichert, wobei die Verbindungshülse (8) an einem proximalen Verbindungsabschnitt (80) ein Verbindungselement (81) aufweist, das zur Verbindung mit einem proximalseitig des Kuppelelements (72) angeordneten Verbindungselement (71) des Außenschafts (7) ausgebildet ist, und wobei die Verbindungshülse (8) distalseitig an einem radialen Absatz (55) der Gabelarme (5), der zwischen dem Armabschnitt (50) und dem Verbindungsabschnitt (51) ausgebildet ist, anschlägt.

15. Chirurgisches Bipolar-Instrument (10), das ein chirurgisches Maulteilwerkzeug (1) mit zwei Maulteilen (2, 2'), einem Betätigungselement (3) und einer Gabelhalterung (4) aufweist, durch die sich das Betätigungselement (3) zum Eingriff mit den zwei Maulteilen (2, 2') erstreckt, die in der Gabelhalterung (4) schwenkbar um zumindest eine Schwenkachse (S) gelagert sind, wobei die Gabelhalterung (4) an einem distalen Ende eines Außenschafts (7) angeordnet ist, durch den sich das Betätigungselement (3) längsbeweglich erstreckt, das mit einer Betätigungseinrichtung (9) in Eingriff steht, die an einem proximalen Ende des Außenschafts (7) angeordnet ist,
**dadurch gekennzeichnet, dass**
das chirurgische Maulteilwerkzeug (1) ein chirurgisches Bipolar-Maulteilwerkzeug (1) nach zumindest einem der Ansprüche 1 bis 14 ist.

16. Verfahren zur Herstellung eines chirurgischen Bipolar-Maulteilwerkzeugs (1) umfassend die Schritte
S0) Bereitstellen eines Montagesets, das zwei Verbundbau-Maulteile (2, 2'), ein Verbundbau-Betätigungselement (3) und eine Gabelhalterung (4), die aus einem Leitermaterial gefertigt ist, umfasst, dabei
Herstellen der Verbundbau-Maulteile (2, 2') und des Verbundbau-Betätigungselements (3) durch
- Fertigen eines ersten Wirkabschnitts (24) aus einem Leitermaterial und Fertigen eines ersten Betätigungsabschnitts (20) aus einem Isolatormaterial und Verbinden des ersten Wirkabschnitts (24) und des ersten Betätigungsabschnitts (20) zu dem ersten Verbundbau-Maulteil (2), und
- Fertigen eines zweiten Wirkabschnitts (24') aus einem Leitermaterial und Fertigen eines zweiten Betätigungsabschnitts (20') aus einem Isolatormaterial und Verbinden des zweiten Wirkabschnitts (24') und des zweiten Betätigungsabschnitts (20') zu dem zweiten Verbundbau-Maulteil (2'), und
- Fertigen des Verbundbau-Betätigungselement (3) aus einem Leitermaterial und einem Isolatormaterial, und
S1) in Eingriff Bringen der beiden Verbundbau-Maulteile (2, 2') mit dem Verbundbau-Betätigungselement (3) und Lagern der Verbundbau-Maulteile (2, 2') in der Gabelhalterung (4), dabei
- elektrisches Verbinden des ersten Wirkabschnitts (24) des ersten Verbundbau-Maulteils (2) mit dem Verbundbau-Betätigungselement (3) und elektrisches Isolieren des ersten Wirkabschnitts (24) von der Gabelhalterung (4) durch den ersten Betätigungsabschnitt (20), und
- elektrisches Verbinden des zweiten Wirkabschnitts (24') des zweiten Verbundbau-Maulteils (2') mit der Gabelhalterung (4) und elektrisches Isolieren des zweiten Wirkabschnitts (24') von dem Verbundbau-Betätigungselement (3) durch den zweiten Betätigungsabschnitt (20'), und
- elektrisches Isolieren des Verbundbau-Betätigungselements (3) und der Gabelhalterung (4) voneinander.
